# EUROPEAN PATENT SPECIFICATION

(11) **EP 3 273 890 B1**
(45) Date of publication and mention of the grant of the patent: **22.07.2020**
(21) Application number: 16767851.5
(22) Date of filing: 24.03.2016
(51) Int. Cl.: A61B 18/12, A61B 18/14, A61B 18/00, A61B 18/16, H01H 23/28, H01H 36/00

(54) **ELECTROSURGICAL SWITCH ASSEMBLY AND RELATED SYSTEMS**
ELEKTROCHIRURGISCHE SCHALTERANORDNUNG SOWIE ZUGEHÖRIGE SYSTEME
MONTAGE DE COMMUTATEUR ÉLECTROCHIRURGICAL ET SYSTÈMES

(30) Priority: 24.03.2015 US 201562137806 P
(43) Date of publication of application: 31.01.2018
(73) Proprietor: Weber, Paul, Palm Harbor, Florida 34683 (US)
(72) Inventor: WEBER, Paul, Palm Harbor, Florida 34685 (US); KANE, Mark, Palm Harbor, Florida 346 (US)
(74) Representative: Lock, Howard John
(86) International application number: PCT/IB2016/051682
(87) International publication number: WO 2016/151526

(56) References cited:
- WO-A1-2014/207640
- US-A- 6 113 596
- US-A1- 2005 113 820
- US-A1- 2008 004 619
- US-A1- 2009 093 804
- US-A1- 2009 248 007
- US-A1- 2011 144 729
- US-A1- 2014 018 795
- US-B2- 6 652 514

## Description

### FIELD OF THE INVENTION

This invention relates to electrosurgical devices.

### BACKGROUND OF THE INVENTION

US 2009/0248007 A1 discloses an electrosurgical system comprising an electrosurgical generator, a feedback circuit or controller, and an electrosurgical tool.

US 2005/0113820 A1 discloses an electrosurgical generator for generating radio frequency power. The generator comprises a radio frequency output stage having three or more output connections, one or more sources of output power coupled to the output stage, and a controller operable to cause the generator to supply a first cutting RF waveform to the output connections or a second coagulating RF waveform to the output connections.

US 2009/093804 A1 discloses an electrosurgical system which includes a generator for generating radio frequency power, an electrosurgical instrument including at least first and second electrodes carried on the instrument, and a monopolar patient return electrode separate from the instrument.

WO 2014/207640 discloses apparatus for tissue separation and/or modification. In some embodiments, the apparatus comprises a tip comprising a plurality of protrusions and at least one lysing element positioned between at least two adjacent protrusions.

### GENERAL DESCRIPTION OF THE INVENTION

According to one aspect of this invention, there is provided an electrosurgical device, comprising:
a first electrode set configured to deliver CUT radiofrequency energy;
a second electrode set configured to deliver COAG radiofrequency energy; and
a switch assembly configured to allow for selection between at least three modes to facilitate operation of the electrosurgical device in the at least three modes, the at least three modes comprising:
   a first, neutral mode, in which the electrosurgical device is configured such that no radiofrequency energy is delivered to either the first electrode set or the second electrode assembly;
   a second, CUT mode, in which the electrosurgical device is configured such that CUT radiofrequency energy may be delivered to the first electrode set through the switch assembly; and
   a third, COAG mode, in which the electrosurgical device is configured such that COAG radiofrequency energy may be delivered to the second electrode set through the switch assembly;
wherein the switch assembly is further configured to allow for selection of a first sub-mode of the CUT mode in which an electrical path between the CUT electrode set and a radiofrequency source is closed but an electrical path associated with a signal circuit for activating the radiofrequency source is open.

### BRIEF DESCRIPTION OF THE DRAWINGS

The written disclosure herein describes illustrative embodiments that are non-limiting and non-exhaustive. Reference is made to certain of such illustrative embodiments that are depicted in the figures, in which:
FIG. 1A is a perspective view of the Tissue Dissector and Modifier and the electrosurgical system thereto.
FIG. 1B is a close-up, perspective view of a Tissue Dissector and Modifier tip and its components.
FIG. 2A is a side perspective view of a switch assembly according to one embodiment.
FIG. 2B is a lower perspective view of the switch assembly of FIG. 2A.
FIG. 2C is a side elevation view of a switch assembly in the neutral setting.
FIG. 2D is a side elevation view of a switch assembly in the CUT setting #1.
FIG. 2E is a side elevation view of a switch assembly in the CUT setting #2.
FIG. 3A is a side perspective view of another embodiment of a switch assembly.
FIG. 3B is a lower perspective view of the switch assembly of FIG. 3A.
FIG. 3C is a perspective view of switch assembly with cutaway view of a spring component.
FIG. 3D is a side elevation view of a switch assembly in the neutral setting.
FIG. 3E is a side elevation view of a switch assembly in the CUT setting #1.
FIG. 3F is a side elevation view of a switch assembly in the CUT setting #2.
FIG. 4A is a side perspective view of another embodiment of a switch assembly in the neutral setting.
FIG. 4B is a lower perspective view of the switch assembly of FIG. 4A.
FIG. 4C is a side elevation view of a switch assembly in the CUT setting #1.
FIG. 4D is a side elevation view of a switch assembly in the CUT setting #2.
FIG. 5A is a side perspective view of yet another embodiment of a switch assembly illustrate with part of the rocker cut away to reveal a contact bridge of the switch assembly.
FIG. 5B is a lower perspective view of the switch assembly of FIG. 5A.
FIG. 5C is a side elevation view of the switch assembly in a neutral setting.
FIG. 5D is a side elevation view of the switch assembly in a first CUT setting.
FIG. 5E is a side elevation view of the switch assembly in a second CUT setting.
FIG. 6A is an upper plan view of a TDM comprising still another embodiment of a switch assembly.
FIG. 6B is a perspective view of the TDM of FIG. 6A.
FIG. 6C is a schematic diagram illustrating one of two actuators of the switch assembly of FIGS. 6A and 6B.
FIG. 7A is a schematic diagram illustrating the functionality of a switch assembly for use in an electrosurgical device according to some embodiments.
FIG. 7B is a schematic diagram of a TDM comprising a temperature sensor according to some embodiments.
FIG. 7C is a schematic diagram illustrating the functional interaction between a switch assembly and a temperature sensor within a TDM according to some embodiments.
FIG. 8 is a schematic diagram illustrating the functionality of another embodiment of a temperature sensor and a switch assembly within a TDM.
FIG. 9A is a perspective view of another embodiment of a bipolar TDM.
FIG. 9B is a close-up view of a tip of the bipolar TDM of FIG. 9A.
FIG. 9C is a schematic diagram illustrating the functionality of a switch assembly and various related components that may be used in connection with the bipolar TDM of FIG. 9A.
FIG. 9D illustrates the CUT electrodes of the bipolar TDM of FIG. 9A shown removed from the tip of the TDM.
FIG. 9E illustrates the COAG electrodes of the bipolar TDM of FIG. 9A shown removed from the tip of the TDM.
FIG. 10A is a side elevation view of an embodiment of a laparoscopic surgical instrument comprising a TDM tip and a spot coagulator.
FIG. 10B is a close-up view of the TDM tip of the surgical instrument of FIG. 10A.
FIG. 10C illustrates an embodiment of a robotic surgery system comprising a flexible shaft and a TDM tip.

### DETAILED DESCRIPTION

Electrosurgery was invented around 1926 by William T. Bovie and is commonly used in surgery today for dissection and coagulation of tissues using different energy waveforms from the radiofrequency (RF) spectrum.

An electrosurgical system is comprised of multiple components. A first electrosurgical system component may comprise the electrosurgical generator (ESG) that connects into an electrical power outlet and converts electrical energy to various RF energy waveforms. An incomplete list of examples of ESGs include Covidien's Valleylab Force series or Bovie Medical Corporation's ICON GP.

A second electrosurgical system component may comprise a means for delivering RF energy from an ESG to patient tissues (an "energy application means"). According to IEC-60601-1, this component may also be defined as an "Applied Part" as this portion of the system is 'applied' to the patient. Examples of energy application means include, for example, electrosurgical pencils that may contain two sub-components: (1) the 'Handpiece' sub- component that may be held in the surgeon's hand, connects to the ESG, may permit surgeon control of one or more RF energies via myriad types of switching mechanisms, and may contain a retention mechanism for (2) various interchangeable metal electrodes that may come in direct or near direct patient contact depending upon the desired therapeutic effect. Another example of an energy application means is a Tissue Dissection and Modification Wand ("TDM"), which is discussed in greater detail below.

A third system component that is typically utilized in monopolar systems, not bipolar systems, may be the dispersive electrode (or "return pad") that is attached to the patient and connects to the ESG thus permitting the RF source circuit to be completed; this creates a return path for the RF energy to the ESG preventing patient burns at the return point.

Many ESGs have been designed to operate in at least two modes: the "CUT Mode" and the "COAG Mode".

The COAG Mode delivers an RF waveform through the Handpiece to the electrode tip that desiccates tissues in the immediate vicinity of the tip containing liquid thus promoting a coagulation or bleeding control effect. Some COAG waveforms require electrode contact with the patient while others require the electrode to be nearby the tissue in order to "spray" the current to nearby tissues. These waveforms require very high currents and voltages, for example, the Bovie ICON GP ESG in its instructions for use state the following (in Watts (peak to peak) and Volts): Pinpoint mode, 120W, 4000V; Spray mode, 120W, 7000V.

The primary purpose of the CUT Mode is to electrically cut or sever tissue by delivering an RF waveform that electrically arcs from the electrode. As this cutting/arcing waveform is not designed to control bleeding, ESG designers developed the "blended cut" waveform that is one waveform that changes amplitude and frequency and repetition-rate so that it provides a cutting effect as well as a coagulation effect. This Cut/Blend RF Waveform requires very high currents and voltages, for example, the Bovie ICON GP ESG in its instructions for use state the following (in Watts (peak to peak) and Volts): Blend 3, 200W at 2700V. As used herein, the term CUT Mode is intended to be used in its broadest sense to include all possible 'blend' modes that may be available from an ESG.

Manufacturers have designed 3 or more locations in electrosurgical systems for surgeons to activate and/or choose the desired RF waveform. First, at the ESG user interface panel, an assistant may set the desired RF waveform, the wattage, and may activate the chosen waveform on the surgeon's command. Second, surgeons may activate the CUT or COAG Modes pre-set at the ESG user interface via a foot switch. Third, surgeons may activate CUT or COAG Mode via a switch in the Handpiece of the Applied Part and on some models may adjust output wattages on the Handpiece.

Many electrosurgical systems activated at Handpieces and foot switches are designed with 3 circuits. In some ESG models, Applied Parts with 3-pinned plugs connect into a receptacle on the ESG. Circuit 1 may be called the RF source circuit ("Circuit 1/RF Source Circuit") that carries the chosen RF waveform to the electrode(s); this is typically the pin on a standard 3-ping electrosurgical plug that is separate from the other two pins.

The other two pins comprise the second and third circuits that are low voltage signal circuits that are closed when the surgeon (A) chooses the CUT Mode by activating the CUT aspect of the switch (frequently via button or rocker switches) or (B) chooses the COAG Mode by activating the COAG aspect of the switch. Herein, these two circuits will be referred to as Circuit 2/CUT Mode Signal Circuit and Circuit 3/COAG Mode Signal Circuit. Both signal circuits are powered by various means, depending upon the manufacturer, including supplying a low voltage current from the RF Source line.

Many Applied Parts of electrosurgical systems having monopolar outputs are only comprised of one electrode (in or attached to the Applied Part/Handpiece) that carries either the desired cutting and coagulation RF waveforms. Thus, it may be acceptable for such systems to utilize the same electrode or electrodes for both the cutting and coagulation RF waveforms.
However, the Tissue
Dissection and Modification Wand ("TDM"), an Applied Part or energy application means described further herein, utilizes 2 or more separate and distinct electrodes that are components in its distal tip. In some preferred embodiments, one or more Cutting Electrodes and one or more Coagulation Electrodes, which may comprise one or more Energy Window Electrodes, may be built into the TDM's distal tip. Because of the high voltages and waveforms' tendency to arc and the requirement to carry each waveform to a particular electrode, a unique means for switching between the cutting and
coagulation RF waveforms (a "switching means") may be required to operate the TDM effectively and safely. Some embodiments disclosed herein may therefore comprise a unique switch design to be employed in the TDM's handle or at any location along the wiring of the handle leading to the ESG.

The TDM has myriad uses and configurations some of which are described in the following: U.S. Patent No. 6,203,540 titled "Ultrasound and Laser Face-Lift and Bulbous Lysing Device", U.S. Patent No. 6,391,023 titled "Thermal Radiation Facelift Device", U.S. Patent No. 6,432,101 titled "Surgical Device for Performing Face-Lifting Using Electromagnetic Radiation", U.S. Patent No. 6,440,121 titled "Surgical Device For Performing Face-Lifting Surgery Using Radiofrequency Energy", U.S. Patent No. 6,974,450 titled "Face-Lifting Device", and U.S. Patent No. 7,494,488 titled "Facial Tissue Strengthening and Tightening Device and Methods". It has recently been discovered that TDM devices may be utilized many other surgical interventions as well.

Unique to TDM is that it contains at least two separate and distinct electrodes. The Cutting Electrode may be exposed at one or more segments or elements (lysing segments or other lysing elements, for example), each recessed between two bulbous protrusions and designed to deploy the cut or Cut/Blend Waveforms generated during the ESG's CUT Mode. The Coagulation Electrode(s) in such devices may comprise Energy Window Electrode(s), and may terminate at one or more locations on one or more energy windows on the various faces of the TDM (on a top surface in certain preferred embodiments) and may be designed to deploy the coagulation energy waveform produced during the ESG's COAG mode. The term "'modifying" in this context may refer to or may encompass application of energy to tissue using one or more lysing segments or lysing elements of a TDM. In some embodiments the lysing elements used to deliver the CUT or BLEND energy may comprise lysing segments. The term "modifying" in this context may also refer to application of energy to tissue by way of an energy window as described herein.

The TDM may therefore be required to operate with very high voltages and conduct currents in very small and confined spaces. As such, the issue to overcome is that during the activation of either modality (CUT or COAG Modes), one switch end will be connected to a high voltage RF point. High voltage RF energy has potentially dangerous characteristics that must be bridled. First, voltages are high, typically 2000V to 4000V. Thus, any errant current could harm the patient, the user, and/or the equipment internally. Second, RF energy has characteristics that can generate sparks in gaps if not properly isolated. This could immediately damage the device making it unsafe for use, or it could gradually degrade over repeated activations making the device unsafe for use. Finally, the circuitry and switching mechanism must fit in a very small space, in many cases in the space-limited Handpiece.

For the TDM to function safely and effectively with 1-source/2-signal ESG systems, it may be desirable to provide a novel switch or switching assembly (A) when the CUT Mode is activated at the switch by the surgeon and/or to call for Cut/Blend RF Waveform(s) to be created at the ESG and carried through the conductive means to the Cutting Electrode and (B) when the COAG Mode is activated at the switch by the surgeon and/or to call for the Coagulation Waveform to be created at the ESG and channeled through the conductive means to one or more Coagulation Electrodes, such as Energy Window Electrodes.

In addition, given the heat that can be generated around electrosurgical electrodes, in some instances is of value to monitor the temperature of the electrode and/or provide a mechanism to prevent the temperature from exceeding a pre-determined limit.

As used herein, the term "coagulation" should be construed to encompass effects other than strictly coagulative effects, including, for example, any therapeutic effect from heating, including denaturing collagen & elastin, melting fat, disabling nerves and sweat glands. Thus, Coagulation Electrodes, such as Energy Window Electrodes, may be configured to deliver energy designed to perform any of these tissue-altering functions.

In one embodiment, the TDM may comprise two active electrodes and may be configured to allow a user to select from device modes via a means for switching between a plurality of electrosurgical energy modes (a "Switching Means"), which may be positioned in the TDM handle. In some embodiments, the Switching Means may be configured to allow for selection between 3 settings (neutral, CUT mode, and COAG mode). In some such embodiments, the Switching Means may be further configured to provide for 5 possible position configurations within such settings, *i.e.,* Neutral (1 position configuration), CUT Mode (2 position configurations), and COAG Mode (2 position configurations). In some embodiments, it may be important that RF energy not be continuously connected to any circuit when in neutral setting. In the neutral setting, (no button switches pressed), it is preferred that Circuit 1/RF Source Circuit is open and therefore not electrically coupled to any electrode. Likewise, the signal circuits are preferably open in this setting. However, as discussed below, some embodiments are contemplated in which an electrical path to the patient and/or electrode(s) is closed in the neutral mode but a signal circuit to an ESG is open in this mode.

In selecting one electrode in a particular Mode, in some embodiments, the Switching Means may be configured to decrease the possibility that the unselected electrode(s) is energized while selecting the selected electrode(s). In some such embodiments, the Switching Means may be configured to physically decouple the unselected electrode(s) during the process of selecting the other electrode(s). In other embodiments, the Switching Means may be configured to move the unselected electrode away from the Circuit 1/RF Source during the process of selecting the other electrode(s) to reduce the chances of arcing or other similar problems but may not actually physically decouple the unselected electrode (because it may be already physically separated from the RF source in the neutral setting). In some embodiments, the Switching Means may first decouple the signal circuit and then decouple the Circuit 1/RF Source connection to the unselected electrode before closing the selected electrode circuits. The activation of the switch may then first close the electrical connection for the Circuit 1/RF Source to the desired electrode(s) Cutting Electrode(s) or Coagulation Electrode(s) and subsequently close the Circuit 2/CUT Mode Signal or Circuit 3/COAG Mode Signal circuit. In preferred embodiments, this sequence of closing circuits and/or opening or decreasing the possibility of undesired closing of other circuits may be performed automatically due to the structure of the switching assembly.

In some embodiments, the switch assembly or switching means may further be configured to automatically accomplish a desired sequence of deactivation when a particular mode is deselected, such as, for example, when a user lifts his or her finger from a button or switch of a TDM or other electrosurgical device. For example, some embodiments that are configured to make an electrical connection to the patient/electrode first and then subsequently close the path to the ESG to tell it which mode to activate may further be configured to decouple the various electrical paths in a precise sequence opposite to the activation sequence, namely, the ESG path may be opened first during de-activation, after which the path to the patient/electrode may be opened By providing switch assembly or switching means that ensures that the activation, and deactivation, of the electrical paths to the patient and ESG take place sequentially, rather than simultaneously, and in the proper order, arcing/sparking and other such problems may be avoided.

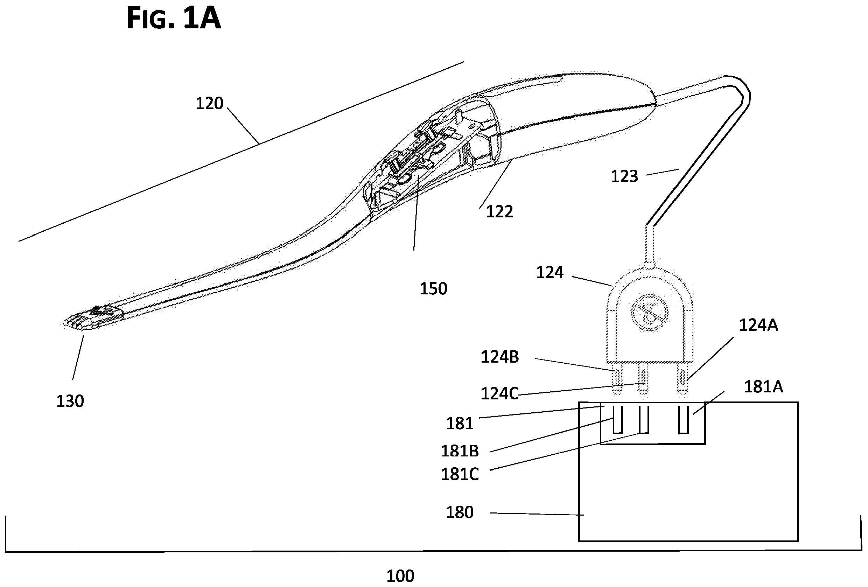

FIG. 1A illustrates a preferred embodiment of an electrosurgical system 100 comprising a switching assembly 150, as described above. In the depicted embodiment, system 100 comprises a TDM system **100** that comprises an ESG **180** and a TDM device **120.** ESG **180** comprises a 3-pinned plug receptacle **181** that comprises pin receptacles **181A, 181B,** and. **181C** Pin receptacle **181A** is for Circuit 1/RF Source Circuit. Pin receptacles **181 B & C** are signal circuits for the CUT & COAG Modes, respectively.

TDM device **120** further comprises tip **130,** handle **122,** source/signal wire **123,** and 3-pinned plug **124.** The 3-pinned plug **124** comprises pins **124A, 124B, and 124C:** Pin **124A** is part of the Circuit 1/RF Source Circuit while pins **124 B & C** are parts of the Circuit 2/CUT Mode Signal Circuit and Circuit 3/COAG Mode Signal Circuit, respectively. The 3-pinned plug **124** may connect into ESG receptacle **181** having corresponding pin receptacles **181A, 181B, and 181C.** Handle **122** may receive source/signal wire **123** and house the switching assembly 150, which is one example of a switching means, as described above.

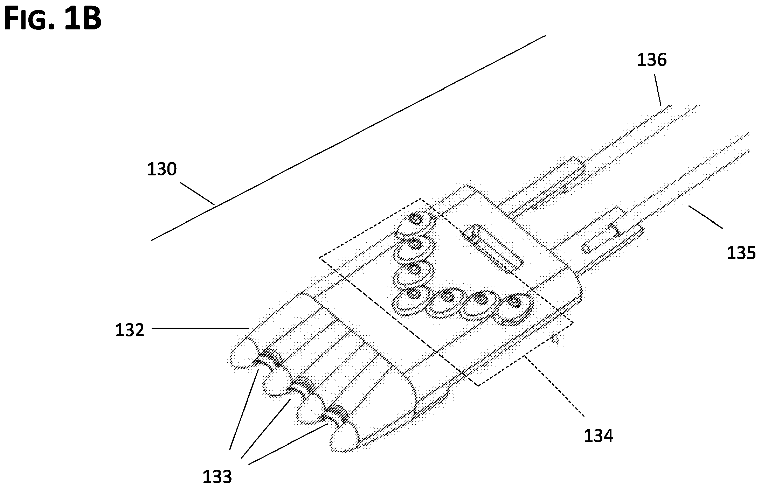

FIG. 1B illustrates tip **130** that may comprise a housing **132,** which may be made up of a ceramic or other preferably non-conductive material. Cutting Electrode **133** may comprise a plurality of segments. Such segments may be positioned in between a plurality of protrusions 131 positioned at the distal end of tip 130. Tip 130 further comprises a plurality of Coagulation electrodes **134** positioned within an Energy Window. Electrodes 134 terminate at 7 termini atop 7 apices in a chevron configuration. Cutting electrode wire lead **135** and energy window electrode wire lead **136** extend from a proximal end of tip 130. Housing **132** may comprise one or more bulbous protrusions 131 between which are located the recessed segments of Cutting Electrode **133** that distribute Cut/Blend RF waveforms.

In order to prevent arcing, sparking, or other unwanted electrical events, the Cutting Electrode(s) and Coagulation Electrode(s) are preferably separated dielectrically when coming in close proximity using dielectric materials, for example, Kapton® material and/or high temperature epoxies.

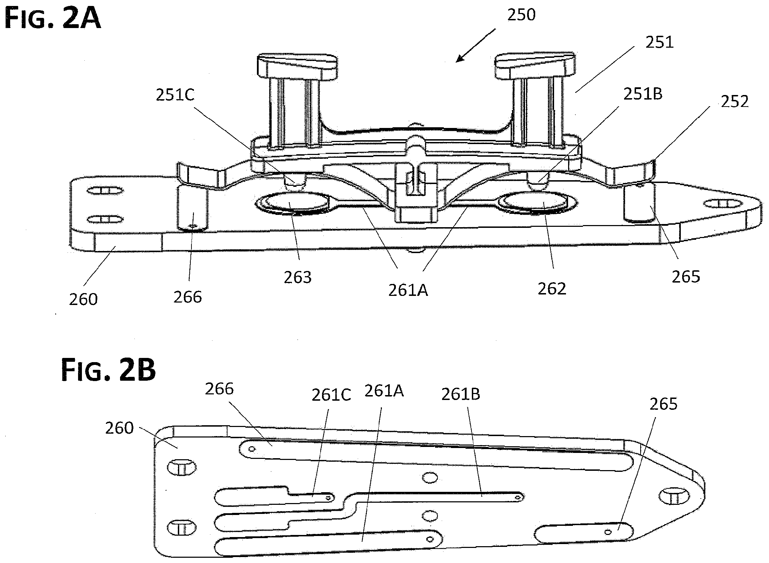

Figs. 2A and 2B illustrate an embodiment of a switching assembly 250 comprising one example of a switching means for switching between cutting and coagulation RF waveforms. Switching assembly **250** comprises rocker **251,** contact spring **252,** and switchboard **260.** Switchboard **260** comprises RF Source Circuit trace **261A,** CUT Mode signal trace **261B,** and COAG Mode Signal trace **261C.** Switchboard **260** further comprises Cut or Blend RF output trace **265** and COAG/Energy Window RF output trace **266.** Switchboard **260** further comprises two dome switches **262** and **263,** each of which is configured to close a signal circuit (CUT and COAG, respectively) when depressed by one of the two plungers **251B** (CUT) or **251C** (COAG) of rocker **251.** Contact spring **252** may be coupled to switchboard 260 atop RF Source Circuit input trace **261A** and thus carries the RF energy to the appropriate electrode path.

Because of the characteristics of high voltage RF energy, it is not a preferred method to activate each specific mode in one step. Otherwise, potentially dangerous arcing and sparking may occur. For patient and user safety, as well as the durability of the TDM device or other electrosurgical device, it is preferred to employ two steps for each mode activation: (1) to complete the path from the RF source line to the chosen electrode and then (2) to signal the ESG to activate and deliver the chosen RF energy to the patient via the path defined by the RF source lead, the switch assembly **250** and its traces, the specific wire leading to the desired CUT or COAG electrode(s), and the electrode. In some embodiments, at least 3mm of clearances may be provided between all traces and current carrying components. Thus, in some embodiments, at least a 3mm clearance may be provided between an output trace associated with an unselected electrode before contact is made with an output trace of a selected electrode. In some embodiments, this clearance may be provided in a neutral setting. Alternatively, this clearance may only be provided upon activation of a selected electrode during operation of the switching assembly **250.** Thus, in some embodiments, a clearance, but an insufficient clearance, may be provided in a neutral setting and this clearance distance may be increased during actuation of switching assembly **250.** Alternatively, no clearance may be provided in a neutral setting and sufficient clearance may be provided during actuation of switching assembly **250,** as described in greater detail below.

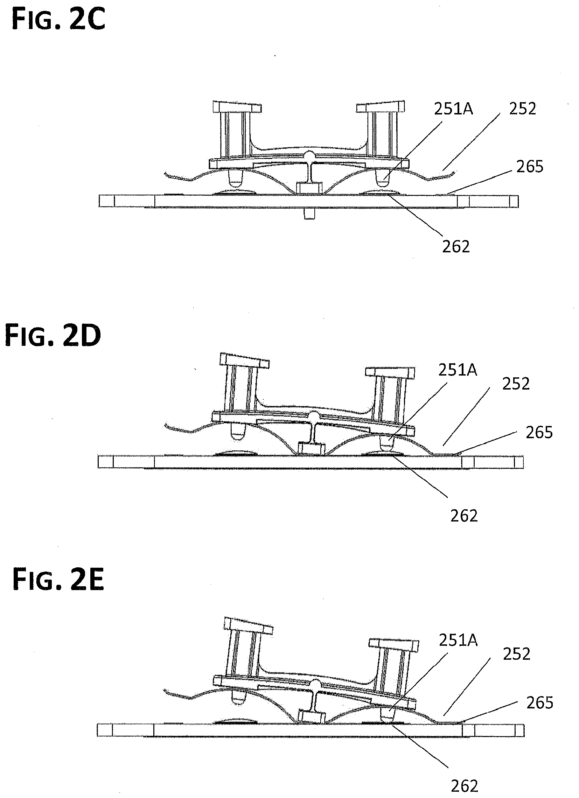

Switch assembly **250** may be configured to operate using 5 settings, one of which may be a default or neutral setting not requiring any user interaction, two of which may be sequentially selected during operation of switch assembly **250** in one direction and/or with respect to one primary mode of operation (CUT or COAG), and the other two of which may be sequentially selected during operation of switch assembly **250** in the other direction and/or with respect to the other primary mode of operation. More particularly, switch assembly **250** may be configured to operate in the following settings/positions:
1) Neutral in which no signal circuit is closed (no plunger **(251B** or **C** is depressing a dome switch **262** or **263).** In some cases (including the embodiment depicted in FIG. 2A), neutral may further mean that no RF path to an electrode is made (by each foot of the contact spring having sufficient clearance from RF output path traces **265** and **266** beneath them). Fig. 2C further illustrates the neutral position in that plunger **251A** is not depressing dome switch **262** and contact spring **252** is not making contact with Cut/Blend RF output trace **265.**
2) CUT Setting 1 in which the RF path to the Cutting Electrode is made (contact spring **252** contacts Cut/Blend RF output trace **265**) but Circuit 2/CUT Mode Signal Circuit is not yet closed (dome switch **262** has not yet been depressed by plunger **251B**). Fig. 2D further illustrates the CUT Setting 1 position in that plunger **251A** is not depressing dome switch **262** but contact spring **252** is making contact with Cut/Blend RF output trace **265.** The depicted embodiment is configured to automatically transition to CUT Setting 1 upon depressing the button of rocker **251** corresponding with (atop) plunger **251A** and, as discussed below, to transition automatically to CUT setting 2 upon further depression of this button.
3) CUT Setting 2 in which the RF path to the Cutting Electrode remains made and Circuit 2/CUT Mode Signal Circuit is closed by depressing dome switch 262 with plunger 251A, thereby causing the ESG to generate and send Cut or Blend RF energy through the RF Cut Circuit to the Cutting Electrode. Fig. 2E illustrates the CUT Setting 2 position in that plunger **251A** is depressing dome switch **262** after contact spring **252** has made contact with Cut/Blend RF output trace **265.** As mentioned above, the depicted embodiment, and other embodiments depicted herein, is configured to allow for making this important transition of electrical contacts simply by pressing on one side of rocker **251.**
4) COAG Setting 1 in which the RF path to the Coagulation Electrode (in this case, the Energy Window Electrode) is made but Circuit 3/COAG Mode Signal Circuit is not yet closed. This position is not illustrated but is the mirror image of Figure 2D on the left side of the switch assembly **250** from the perspective of the figure.
5) COAG Setting 2 in which the RF path to the Energy Window Electrode remains made and Circuit 3/COAG Mode Signal Circuit is closed, thereby causing the ESG to generate Coagulation RF Waveforms that flow through the RF COAG circuit to the Energy Window Electrode. This position is not illustrated but is the mirror of FIG. 2E on the left side of the switch assembly **250** from the perspective of the figure.

Some embodiments may further be configured such that the switch assembly 250 is configured to perform the precise sequence described above in reverse when a particular electrode activation mode is being disabled or turned off. For example, when a user removes a force on one side of switch assembly **250** (say, the CUT side), the Circuit 2/CUT Mode Signal Circuit may first be opened by releasing plunger **251A** from dome switch **262.** Switch assembly **250** may be configured to subsequently open the RF path to the Cutting Electrode(s) and/or patient by lifting contact spring **252** from Cut/Blend RF output trace **265.** The same may be true with respect to the opposite side/mode. As mentioned elsewhere herein, switch assembly **250** (or any of the other switch assemblies or switching means disclosed herein) may be configured to allow for this precise, sequential activation, and deactivation, of these electrical paths/circuits automatically simply by depressing a button, switch, or the like and, similarly, releasing the button, switch, or the like.

Notwithstanding the foregoing, it is contemplated that, for use in connection with certain electrosurgical devices and/or for certain applications, it may be acceptable to provide a switch assembly or switching means that is configured to operate in essentially three modes rather than five. More particularly, in some embodiments, any of the switch assemblies disclosed herein may be modified such that depressing a particular button, switch, or portion of a button/switch/etc. associated with a particular mode may result in simultaneous, or at least substantially simultaneous, closing of both the path to the patient and/or electrode(s) and the signal path to the ESG for both of the respective modes/electrodes. Preferably, a neutral mode is still provided between the other two modes.

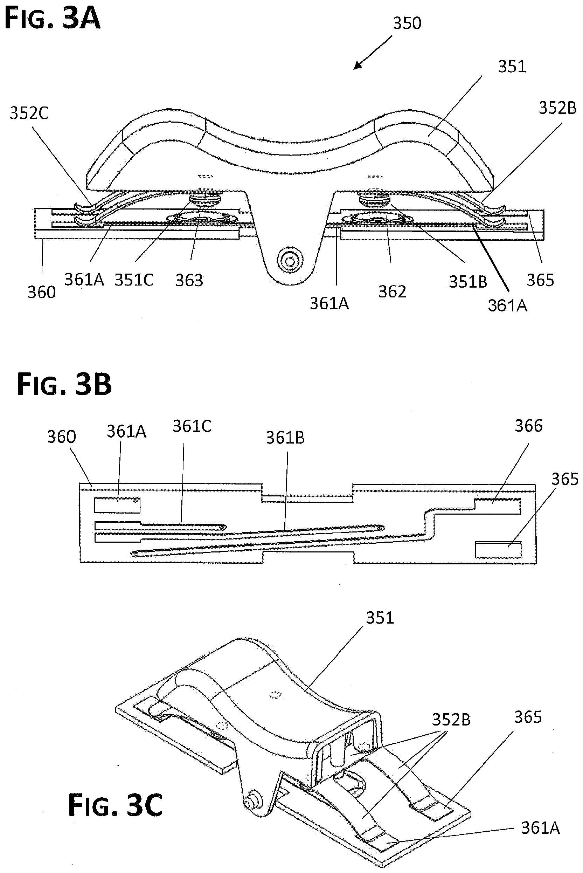

Figs. 3A and 3B illustrate another embodiment of a switch assembly **350** that may be used in a TDM system or another electrosurgical device or system. Switch assembly 350 is another example of a means for means for switching between a plurality of electrosurgical energy modes. Switch assembly **350** comprises rocker **351,** CUT Contact Spring **352B,** COAG contact spring **352C,** and switchboard **360.**

Switchboard **360** comprises RF Source trace **361A,** CUT Mode Signal trace **361B,** and COAG Mode Signal trace **361C.** Switchboard **360** further comprises Cut or Blend RF output trace **365** and COAG/Energy Window RF output trace **366.** Switchboard **360** further comprises two dome switches **362** and **363,** each of which is configured to close a signal circuit (CUT or BLEND and COAG, respectively) when depressed by one of the two plungers **351B** (CUT or BLEND) or **351C** (COAG) of rocker **351.** CUT contact spring **352B** is configured to complete a path between the RF Source trace **361A** and Cut/blend RF output trace **365.** COAG contact spring **352C** is configured to complete a path between the RF Source trace **361A** and COAG RF output trace **366.**

Fig. 3C illustrates how CUT contact spring **352B** may comprise one piece at least substantially in the shape of a "U" when viewed from a top plan view perspective. This U shape, however, may curve from an elevation view perspective to allow for contacting one or more RF source traces into the rocker structure **351.** A similar U-shaped shape or other similar shape may be provided on the opposite side if desired for the other electrosurgical mode traces.

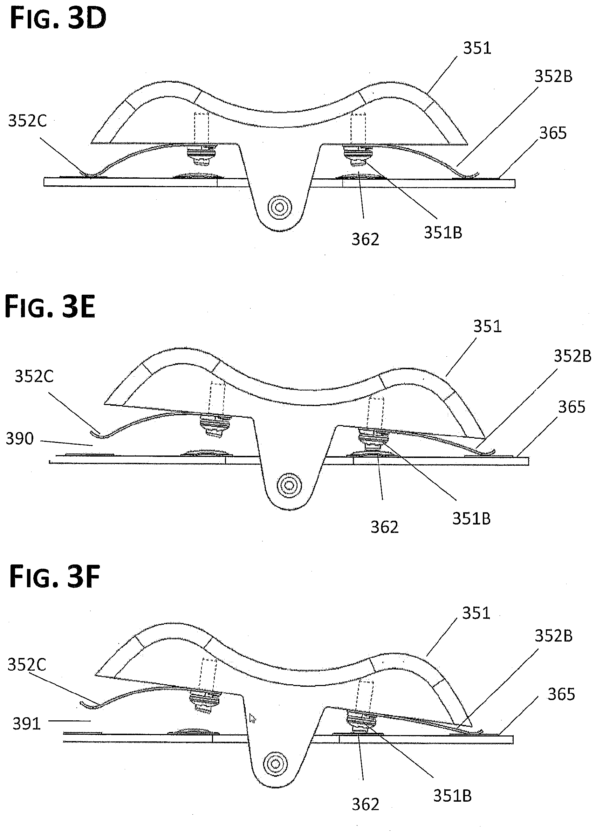

Switch assembly **350** may be configured to operate using 5 settings, one of which may be a default or neutral setting not requiring any user interaction, two of which may be sequentially selected during operation of switch assembly **350** in one direction and/or with respect to one primary mode of operation (CUT or COAG), and the other two of which may be sequentially selected during operation of switch assembly **350** in the other direction and/or with respect to the other primary mode of operation. Unlike switch assembly **250,** however, switch assembly **350** is configured such that, in the neutral setting, the opposing contact springs **352B** and **352C** are configured to be in contact with their respective RF source traces. More particularly, switch assembly **350** may be configured to operate in the following settings/positions:
1) Neutral, in which no signal circuit is closed (no plunger **351 B or C** is depressing a dome switch **362 or 363**). In this embodiment, however, contact springs **352B** and **352C** are permitted to contact the RF source and RF carry-away traces in the neutral setting because the contact between contact springs 352B and 352C and their respective traces in this configuration serve to center and position the rocker mechanism flat when the user is not depressing either end of the rocker. Fig. 3D further illustrates the neutral setting in that plunger **351 B** is not depressing dome switch **362** (nor is the opposite plunger depressing its respective dome switch).
2) Fig. 3E illustrates CUT Setting 1 in which (A) the RF path to the Cutting Electrode continues to be made (contact spring **352B** contacts RF source trace **361A** (visible) and Cut/Blend RF output trace **365** (not visible, hidden from view)), (B) however, COAG contact spring **352C** breaks any contact with RF source trace **361A** and COAG/Energy Window RF output trace **366,** as depicted in FIG. 3D, thereby creating a clearance that is preferably sufficient to avoid arcing between the conductive components, and (C) the Circuit 2/CUT Mode Signal Circuit is not yet closed (dome switch **362** has not yet been depressed by plunger **351B**). Fig. 3E further illustrates the CUT Setting 1 position in that plunger **351 B** is not depressing dome switch **362** and clearance **390** is visible as **352C** lifts away from the traces beneath it.
3) Fig. 3F illustrates CUT Setting 2 in which (A) the RF path to the Cutting Electrode remains closed, (B) the CUT Mode Signal Circuit is also closed, thereby causing the ESG to send Cut or Blend RF Waveforms through the RF CUT circuit to the Cutting Electrode, and (C) COAG Contact Spring **352C** reaches a minimum clearance preferably sufficient to avoid arcing, sparking, or other undesired electrical events. In some embodiments, this clearance may be at least 3 mm. Fig. 3F illustrates the CUT Setting 2 position in which plunger **351 B** has depressed dome switch **362** and contact spring **352B** continues making contact between RF Source trace **361A** and the Cut/Blend RF output trace **365.** Maximum clearance, as shown at **391,** is achieved in this figure, which may be 3mm or more. In the embodiment of assembly **350,** the device may be configured such that the sequence depicted in FIGS. 3D-3F, along with a related
   sequence in an opposite direction (not shown in the drawings) may take place automatically upon depressing one side (or the other) of the top of rocker **351.**
4) COAG Setting 1 in which (A) the RF path to the Coagulation/Energy Window Electrode(s) remains closed, (B) the RF path to the Cutting Electrode is open, but (C) Circuit 3/COAG Mode Signal Circuit is not yet closed. This position is not illustrated but is the mirror image of Figure 3E on the opposite end of the switch assembly.
5) COAG Setting 2 in which (A) the RF path to the Coagulation/Energy Window Electrode(s) remains closed, (B) the RF path to the Cutting Electrode remains open, but (C) Circuit 3/COAG Mode Signal Circuit is now closed, thereby causing the ESG to send Coagulation RF Waveforms through the RF COAG circuit to the Coagulation/Energy Window Electrode(s). This position is not illustrated but is the mirror image of FIG. 3F but on the opposite end of the switch assembly.

Some embodiments may further be configured such that the switch assembly 350 is configured to perform the precise sequence described above in reverse when a particular electrode activation mode is being disabled or turned off. For example, when a user removes a force on one side of switch assembly **350** (say, the CUT side), the Circuit 2/CUT Mode Signal Circuit may first be opened by releasing dome switch **362** from plunger **351B.** Switch assembly **350** may be configured to subsequently close the RF path to the COAG Electrode(s) and/or patient on the opposite side. The RF path to the selected electrode(s) (CUT in the scenario described above) remains closed during the entire operation between the neutral and CUT mode. As mentioned elsewhere herein, switch assembly **350** (or any of the other switch assemblies or switching means disclosed herein) may be configured to allow for this precise, sequential activation, and deactivation, of these electrical paths/circuits automatically simply by depressing a button, switch, or the like and, similarly, releasing the button, switch, or the like.

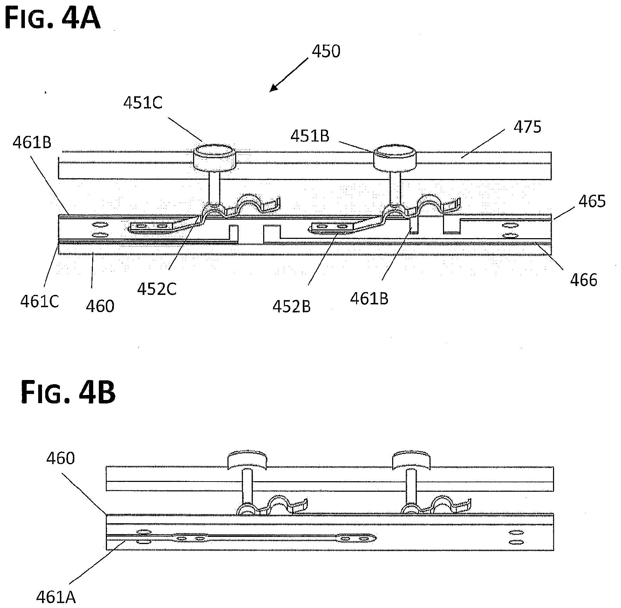

Figs. 4A and 4B illustrate another embodiment of a switch assembly **450.** Switch assembly 450 is also another example of a switching means for switching between cutting and coagulation RF waveforms that may be used in a TDM or another electrosurgical system. Switch assembly **450** comprises outer housing **475,** CUT contact spring **452B,** COAG contact spring **452C,** CUT plunger **451B,** COAG plunger **451C,** and switchboard **460.**

Switchboard **460** comprises RF Source trace **461A,** CUT Signal trace **461B,** and COAG Signal trace **461C.** Switchboard **460** further comprises Cut/blend RF output trace **465** and Coagulation/Energy Window RF output trace **466.** CUT contact spring **452B** is affixed atop RF Source trace **461A** and thus will carry the Cut/Blend RF Waveform current to Cutting Electrode path when the ESG is so activated. Similarly, COAG contact spring **452C** is also affixed atop RF Source trace **461A** and thus will carry the Coagulation RF Waveform to the Coagulation/Energy Window Electrode path when the ESG is so activated.

Switch assembly **450** may be configured to operate in the following settings/positions:
1) Neutral in which no signal circuit is closed (no plunger (**451B** or **451C**) is pressing down on either spring **452B** or **452C**), thus no RF path to an electrode is closed by any pad of either CUT contact spring **452B** or COAG contact spring **452C,** and preferably each contact spring has sufficient clearance from RF output path traces **465** and **466** beneath them to avoid arcing, sparking, etc.

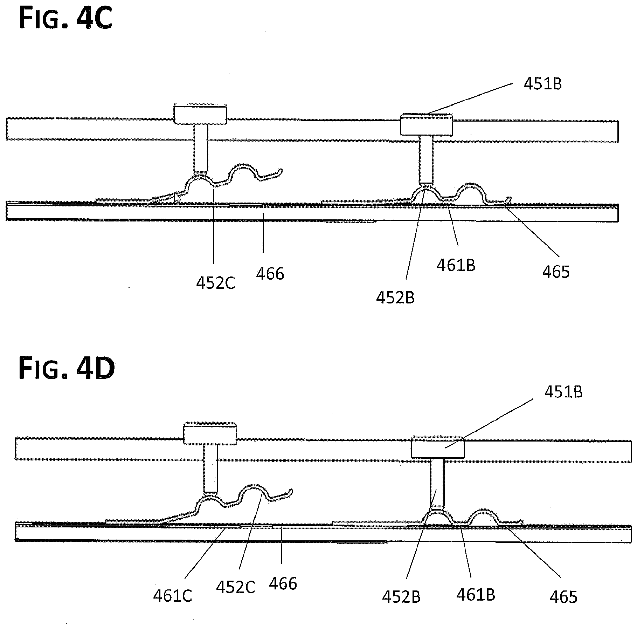

2) Fig. 4C illustrates CUT Setting 1 in which (A) the RF path to the Cutting Electrode is closed (plunger **451B** sufficiently depresses contact spring **452B** so that it contacts Cut/Blend RF output trace **465**) but (B) the Circuit 2/CUT Mode Signal Circuit is not yet closed because CUT contact spring **452B** is not yet contacting CUT Mode signal trace **461B,** and (C) COAG contact spring **452C** continues to have sufficient clearance away from the Coagulation/Energy Window RF trace **466** to avoid acing, sparking, etc. In some embodiments, one or both of contact springs 452B and 452C may be bent or otherwise shaped to facilitate closing the RF path(s) before the respective signal circuit paths. Alternatively, or additionally, the respective signal contacts may be shaped and/or positioned to facilitate the desired staging of these electrical connections.
3) Fig. 4D illustrates CUT Position 2 in which (A) the RF path to the Cutting Electrode remains closed, as mentioned in the previous paragraph, but in which (B) CUT contact spring **452B** makes contact with CUT signal trace **461B,** which completes the circuit to the ESG causing the ESG to deliver a Cut/Blend RF Waveform to the RF source line and eventually to the Cutting Electrode via CUT contact spring **452B.** Preferably, COAG contact spring **452C** continues to have sufficient clearance away from the Energy Window RF output trace **466** to eliminate arcing and/or sparking, etc.
4) COAG Position 1 in which (A) the RF path to the Coagulation/Energy Window Electrode(s) is closed but (B) Circuit 3/COAG Mode Signal Circuit is not yet closed. This position is not illustrated in the drawings. However, the positions of springs **452C** and **452B** in Fig. 4C would be reversed.
5) COAG Position 2 in which (A) the RF path to the Coagulation/Energy Window Electrode(s) remains closed and (B) the pad on COAG contact spring **452C** has been moved downward sufficiently to contact the COAG Mode signal trace **461C** thus causing Circuit 3/COAG Mode Signal Circuit to close and causing the ESG to send Coagulation RF Waveforms through the RF COAG circuit to the Coagulation/Energy Window Electrode(s) via COAG contact spring **452C.** This position is not illustrated but is the mirror image of FIG. 4D but on the left side of the switch assembly. In other words, the positions of springs **452B** and **452C** in Fig. 4D would be reversed for COAG position 2.

In alternative embodiments, each spring/plunger/trace combination may be positioned on a separate switchboard.

Some embodiments may further be configured such that the switch assembly 450 is configured to perform the precise sequence described above in reverse when a particular electrode activation mode is being disabled or turned off. For example, when a user removes a force on one of the buttons/plungers of switch assembly 450 (say, plunger **451B**), the Circuit 2/CUT Mode Signal Circuit may first be opened by releasing CUT contact spring **452B** from CUT signal trace **461 B.** Switch assembly 450 may be configured to subsequently open the RF path to the Cutting Electrode(s) and/or patient by lifting contact spring **452B** from Cut/Blend RF output trace **465.** The same may be true with respect to the opposite side/mode. This effect may be provided for by designing suitable bends into the two contact springs. As mentioned elsewhere herein, switch assembly 450 (or any of the other switch assemblies or switching means disclosed herein) may be configured to allow for this precise, sequential activation, and deactivation, of these electrical paths/circuits automatically simply by depressing a button, switch, or the like and, similarly, releasing the button, switch, or the like. In this particular embodiment, both of the two buttons/plungers are separately configured to provide for a desired sequential activation and deactivation of the electrical paths/circuits.

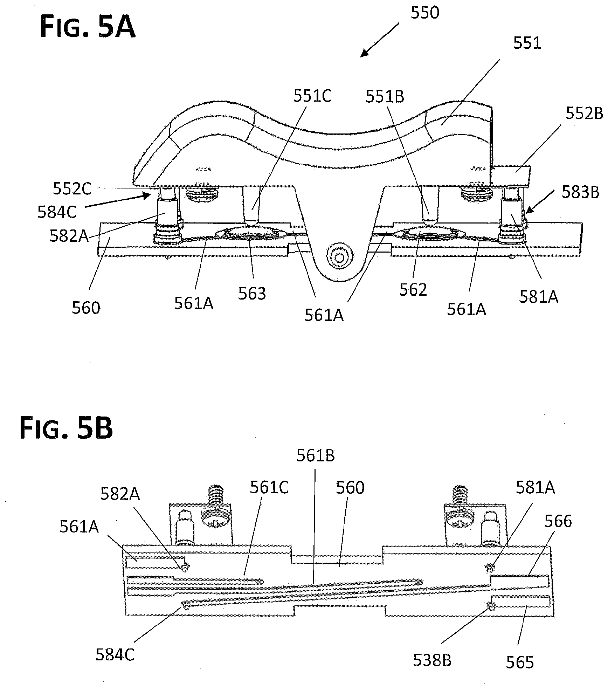

Fig. 5A illustrates another embodiment of a switch assembly **550** that may be used in a TDM system or another electrosurgical device or system. Switch assembly **550** is another example of a means for means for switching between a plurality of electrosurgical energy modes. Switch assembly **550** comprises rocker **551,** CUT Contact Bridge **552B,** COAG contact Bridge **552C,** switchboard **560,** and 4 pogo-pins **581A, 582A, 583B** (behind **581A**), and **584C** (behind 582A). Each pogo-pin may comprise two or more nested cylinders with an internal spring mechanism that returns the pin back to a pre-determined length after compression. Pogo-pins may be conductive and may be capable of making circuits with RF waveforms. Pogo-pins **581A** and **582A** are the RF Source pins to make separate paths to the Cutting Electrode(s) and the Coagulation/Energy Window Electrode(s) for the CUT Mode and the COAG Mode, respectively. Pogo-pin **583B** electrically connects to the electrical pathway of the Cutting Electrode(s). Pogo-pin **584C** electrically connects to the electrical pathway of the COAG/Energy Window Electrode(s).

In Figs. 5A and 5B, switchboard **560** comprises RF Source trace **561A** (connecting both RF Source pogo-pins **581A** and **582A** and dome switches **562** and **563**), CUT Mode Signal trace **561B,** and COAG Mode Signal trace **561C.** Switchboard **560** further comprises Cut or Blend RF output trace **565** and COAG/Energy Window RF output trace **566.** Switchboard **560** further comprises two dome switches **562** and **563,** each of which is configured to close a signal circuit (CUT or BLEND and COAG, respectively) when depressed by one of the two plungers **551B** (CUT or BLEND) or **551C** (COAG) of rocker **551.** CUT Contact Bridge **552B** is configured to electrically connect, upon depression of the CUT Mode side of the rocker, RF Source trace **561A** and Cut/Blend RF output trace **565** via RF Source CUT Pogo-pin **581A** and Cut or Blend RF output Pogo-pin **583B.** COAG Contact Bridge **552C** is configured to electrically connect, upon depression of the other side of the rocker (the COAG Mode rocker side), RF Source trace **561A** and COAG/Energy Window RF output trace **566** via RF Source COAG Pogo-pin **582A** and COAG RF output Pogo-Pin **552C.**

Fig. 5A illustrates how CUT Contact Bridge **552B** may comprise one piece of conductive material substantially in the shape of a rectangle when viewed from a top plan view perspective. This rectangular shape may permit it to be affixed to the rocker on one side while serving as a conductive bridge between the two pogo-pins on the CUT Mode circuit **581A** and **583B.**

A similar rectangular-shaped shape or other similar or suitable shape may be provided on the opposite side if desired for the other electrosurgical mode traces.

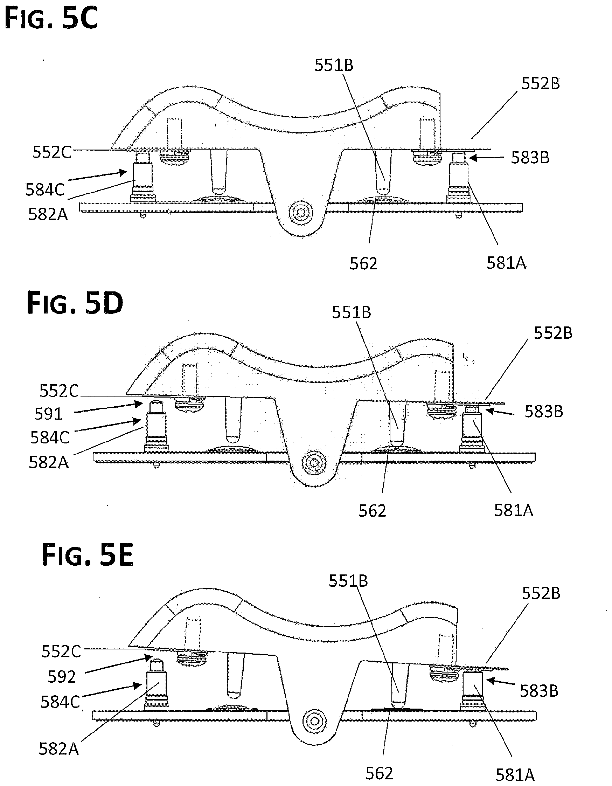

Switch assembly **550** may be configured to operate using 5 settings, one of which may be a default or neutral setting not requiring any user interaction, two of which may be automatically, sequentially selected during operation of switch assembly **550** in one direction and/or with respect to one primary mode of operation (CUT or COAG), and the other two of which may be automatically, sequentially selected during operation of switch assembly **550** in the other direction and/or with respect to the other primary mode of operation. Unlike switch assembly **250,** however, switch assembly **550** is configured such that, in the neutral setting, the opposing contact bridges 552B and 552C are configured to be in contact with their respective RF source traces and signal traces via the respective pogo-pins. More particularly, switch assembly **550** may be configured to operate in the following settings/positions:
1) Neutral, in which no signal circuit is closed (no plunger **551 B or C** is depressing a dome switch **562** or **563**)). In this embodiment, however, contact bridges **552B** and **552C** are permitted to contact the RF source pogo-pins **581A** and **582A** and RF carry-away pogo-pins **583B** and **584C** in the neutral setting because the contact between contact bridges **552B** and **552C** and their respective pogo-pins in this configuration serves to center and position the rocker mechanism flat when the user is not depressing either end of the rocker. Fig. 5C illustrates the neutral setting in that plunger **551 B** is not depressing dome switch **562** (nor is the opposite plunger depressing its respective dome switch).
2) CUT Setting 1 in which (A) the RF path to the Cutting Electrode(s) continues to be made (contact bridge **552B** contacts RF source pogo-pin **581A** and Cut/Blend RF output pogo-pin **583B**), (B) however, COAG contact bridge **552C** breaks any contact with RF source pogo-pin **582A** and COAG/Energy Window RF output pogo-pin **584C,** as depicted in FIG. 5D, thereby creating a clearance **591** that is preferably sufficient to avoid arcing between the metal components (at least 3 mm in some embodiments), and (C) the Circuit 2/CUT Mode Signal Circuit is not yet closed (dome switch **562** has not yet been depressed by plunger **551B**). Fig. 5D illustrates the CUT Setting 1 position in that plunger **551B** is not depressing dome switch **562** and clearance **591** is visible as contact bridge **552C** lifts away from the pogo-pins beneath it.
3) CUT Setting 2 in which (A) the RF path to the Cutting Electrode(s) remains closed by the bridge as disclosed in the previous step, (B) the CUT Mode Signal Circuit is also closed by depression of dome switch **562,** thereby causing the ESG to send Cut or Blend RF Waveforms through the RF CUT circuit through pogo-pin **581A** through contact bridge **583B** through Cut or Blend RF output trace **583B** to Cut or Blend RF output trace **565** to the Cutting Electrode(s), and (C) COAG Contact bridge **552C** reaches a minimum clearance **592,** preferably sufficient to avoid arcing, sparking, or other undesired electrical events. In some embodiments, this clearance may be at least 3 mm. Fig. 5E illustrates the CUT Setting 2 position in which plunger **551B** has depressed dome switch **562** and contact bridge **552B** continues making contact between Cut or Blend RF Source pogo-pin **581A** and the Cut/Blend RF output pogo-pin **583B.** Maximum clearance, as shown at **592,** is achieved in this figure, which may be 3 mm or more. In the embodiment of assembly **550,** the device may be configured such that the sequence depicted in FIGS. 5C-5E, along with a related sequence in an opposite direction (not shown in the drawings) may take place automatically upon depressing one side (or the other) of the top of rocker **551.**
4) COAG Setting 1 in which (A) the RF path to the Coagulation/Energy Window Electrode(s) remains closed, (B) the RF path to the Cutting Electrode is now open, but (C) Circuit 3/COAG Mode Signal Circuit is not yet closed. This position is not illustrated but is the mirror image of Figure 5D on the opposite end of the switch assembly.
5) COAG Setting 2 in which (A) the RF path to the Coagulation/Energy Window Electrode(s) remains closed, (B) the RF path to the Cutting Electrode remains open, but (C) Circuit 3/COAG Mode Signal Circuit is now closed, thereby causing the ESG to send Coagulation RF Waveforms through the RF COAG circuit to the Coagulation/Energy Window Electrode(s). This position is not illustrated but is the mirror image of FIG. 5E but on the opposite end of the switch assembly.

In some embodiments, the switch assembly **550** may be configured to perform the precise sequence described above in reverse when a particular electrode activation mode is being disabled or turned off. For example, when a user removes a force on one side of the rocker of switch assembly **550** (say, the CUT side) after activating and using the CUT mode, the Circuit 2/CUT Mode Signal Circuit may first be opened by releasing plunger **551B** from dome switch **562.** Switch assembly **550** may be configured to subsequently decrease and, ultimately, eliminate clearance **591** and allow COAG contact bridge **552C** to make contact with RF source pogo-pin **582A** and COAG/Energy Window RF output pogo-pin **584C.** As mentioned elsewhere herein, switch assembly **550** (or any of the other switch assemblies or switching means disclosed herein) may be configured to allow for this precise, sequential activation, and deactivation, of these electrical paths/circuits automatically simply by depressing a button, switch, or the like and, similarly, releasing the button, switch, or the like. In this particular embodiment, a particular desired sequence is activated automatically by depressing on one side of the rocker and automatically deactivated in reverse order by releasing pressure on the same side of the rocker.

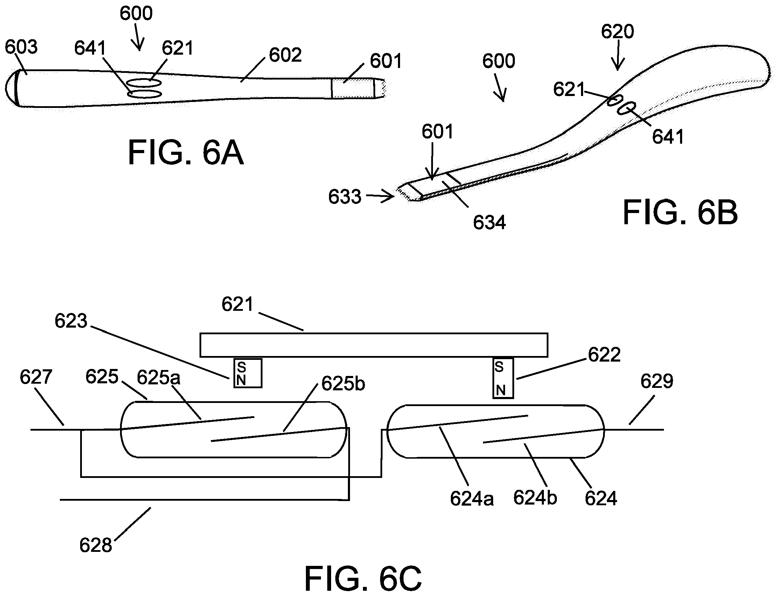

FIGS. 6A and 6B illustrate another embodiment of a TDM 600 comprising yet another alternative embodiment of a switch assembly **620** utilizing reed switches to achieve a safe activation sequence. Reed switches **624** and **625** may comprise glass tubes filled with inert gas and two contacts **624a & 624b** and **625a & 625b** that can be pulled together or apart using a magnetic field. The magnetic field may be generated from, for example, a permanent magnet, a coil formed around the axis of the tube, or other means that generate magnetic fields. FIG. 6C, which illustrates the components of one of the two actuators (actuator **621**) of switch assembly **620,** illustrates one embodiment using permanent magnets **622** and **623** but a coil could also be used if it were provided power from a battery or power scavenging circuit, as described elsewhere in this disclosure.

TDM 600 comprises tip **601,** shaft **602,** and handle **603.** FIG. 6A illustrates tip **601** that may be made up of a ceramic or other preferably non-conductive material. TDM 600 may comprise a cutting electrode set **633** and a COAG electrode set **634,** each of which may comprise one or more electrodes. Cutting electrode set **633** may comprise a plurality of lysing elements. In some embodiments, the plurality of lysing elements may comprise lysing segments. Such lysing elements may be positioned in between a plurality of protrusions positioned at the distal end of tip **601.** Tip **601** further comprises a coagulation electrode set **634,** which may comprise a plurality of coagulation electrodes positioned within an Energy Window. Electrode set **634** may, in some embodiments, terminate at a set of termini atop tip **601** in a chevron configuration. FIG. 6C more particularly illustrates the elements of one actuator or button **621** of switch assembly **620.** Actuator **621** may be configured for activating a CUT mode of TDM **600** and actuator **641** may be configured for activating a COAG mode of TDM **600.** Buttons or actuators **621** and **641** of switch assembly **620** may each comprise two magnets, which may comprise permanent magnets, positioned underneath the respective buttons. With respect to each of the buttons, a first magnet **622** may correspond to the CUT electrode set **633** and second magnet **623** to the ESU CUT signal. Similarly, with respect to actuator **641,** a first magnet (not shown) may correspond to the COAG electrode set 634 and a second magnet (not shown) may correspond to the ESU COAG signal.

RF source in lead **627** may supply both RF energies and the current for signal circuit activation. Magnets **622** and **623** may be oriented such that, as button **621** is depressed, the pole of magnet **622** to the CUT electrode set **633** (or, in the case of button 641, to the COAG electrode set **634**) comes in proximity of the CUT electrode reed switch **624** (and its contacts **624a** & **624b**) before magnet to ESU CUT signal **623** comes close to CUT signal reed **625** (and its contacts **625a** & **625b**), thereby making the patient/electrode circuit first via the CUT electrode line **629.** Configuring magnets **622** and **623** to perform this electrical coupling sequentially may be made possible by using magnets of different sizes, using magnets having different magnetic field strengths, or positioning one of the magnets closer to the patient/electrode circuit than the signal circuit, for example.

As the button continues to travel downward, the pole of magnet **623,** which may be shorter/smaller than magnet **622,** positioned further away from reed switch **625** than magnet **622** is from reed switch **624,** and/or may have a lesser magnetic field strength than magnet **622,** may actuate the CUT signal reed switch **625** and its contacts **625a** & **625b,** thereby completing the CUT signal circuit to the ESU via CUT signal path to ESU **628.** When the button is released, magnet **623** opens reed switch **625,** thereby signaling the ESU to stop delivering CUT energy. As the switch continues to travel upward, magnet **622** finally disconnects the CUT electrode from the active RF by disengaging contacts **624a** & **624b** of reed switch **624.** Thus, the sequence of coupling of the two electrical paths referenced above may take place in reverse sequence upon releasing button **621** or otherwise deactivating a similar actuator. The same or a similar sequence can be applied for COAG modes with a separate COAG electrode set or any other electrode modality. Thus, the sequence described above may also take place when COAG button/actuator **641** is depressed or otherwise actuated and, similarly, may take place in reverse upon deactivating COAG button/actuator **641.** In an additional embodiment, CUT button/actuator **641** may be a part of another structure affecting a COAG or another modality button/actuator in such a way that as one modality is pressed (CUT in this instance), the other magnet(s) move further away from their respective reed switches thus preventing two or more electrodes or electrode sets from carrying RF at the same time and/or with the same modality. Similarly, COAG button **641** may be part of another structure affecting a CUT or another modality button/actuator in such a way that as one modality is pressed (COAG in this instance), the other magnet(s) move further away from their respective reed switches to, again, prevent two distinct electrodes or electrode sets from carrying RF energy at the same time and/or with the same modality.

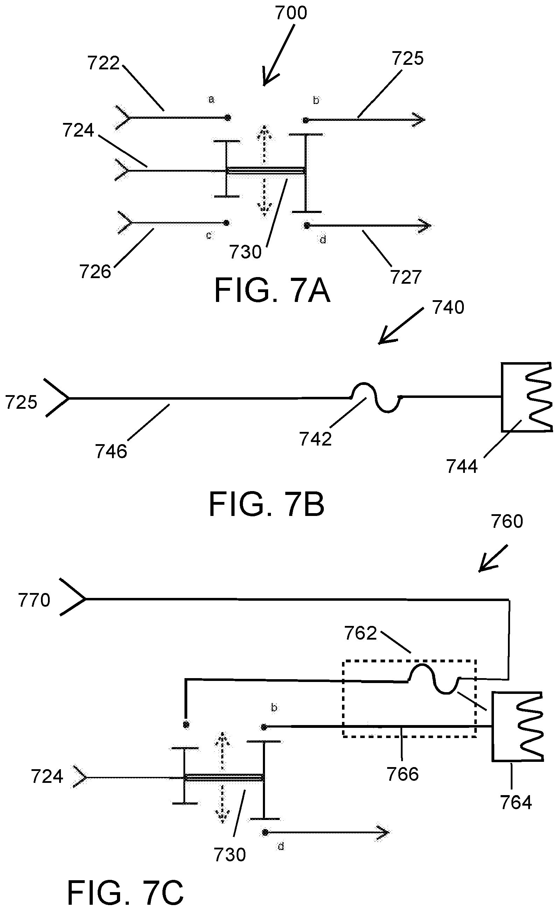

FIG. 7a is a schematic diagram of a switch **700** that may be used to sequentially couple one or more electrodes of a TDM or another electrosurgical device to one or more components of an electrosurgical generator unit (ESU) or another similar unit. The three lines on the left side of the diagram may represent an interface with various typical ports of an ESU. For example, line **722** may be coupled with a "CUT Signal" port of an ESU, line **724** may be configured to interface with an RF IN port, and line **726** may be configured to interface with a "COAG Signal" port. Switch **700** may operate, for example, by moving switch **700** in one of two directions (up and down from the perspective of the figure). By moving the switch **700** up, the structure of switch **700** may be configured to first electrically couple with contact "b", which may complete a circuit between one or more electrodes (preferable CUT electrodes) through line **725** and an RF IN port through line **724.** Continued pressing or other movement of switch **700** in the same direction subsequently may result in electrically coupling with contact "a", which may send a CUT signal to the ESU to turn on the CUT mode.

Similarly, by moving the switch **700** in the opposite direction (down from the perspective of the figure), the structure of switch **700** may be configured to first electrically couple with contact "d", which may complete a circuit between one or more electrodes (preferably COAG electrodes) and an RF port through line **724.** Continued pressing or other movement of switch **700** in the same direction may subsequently result in electrically coupling with contact "c", which may send a COAG signal to the ESU to turn on the COAG mode.

Switch **700** may be physically structured to accomplish making these sequential electrical connections automatically upon actuation. For example, in some embodiments, switch 700 may be configured to pivot or flex once contact is made on one side (e.g., with contact b or d) so as to subsequently make contact with a or c, respectively. This may be accomplished by making the contacts on one side of switch **700** longer, as illustrated in FIG. 7a.

Alternatively, or additionally, switch **700** may comprise one or more flexible materials or components configured to facilitate such sequential coupling. For example, in some embodiments, the central portion of switch **700** in between the opposite ends may comprise a spring **730,** such as a moustache spring, as suggested by the triple-lined area within switch **700,** which may be configured to allow for flexing of the spring as soon as an initial contact is made with contact b or d to then allow for subsequent coupling with contact a or c, respectively.

FIG. 7b depicts an embodiment of an electrosurgical instrument, such as a TDM **740** comprising a thermal fuse **742.** Thermal fuse **742** may be positioned in series with and may also be located in close proximity to, one or more of the electrodes within the tip **744** of TDM **740,** such as one or more cutting electrodes, or in other locations within and/or about the tip **744** and/or shaft **746.** If thermal fuse **742** opens, which may represent an indication that the blade temperature, other electrode temperature, or a temperature of another portion of tip **744** is exceeded, the device and/or one or more modes of the device may be rendered inoperable. For example, if thermal fuse **742** is coupled with a CUT electrode, the CUT mode may be rendered inoperable upon opening of thermal fuse **742.** In other contemplated embodiments the thermal fuse **742** may be coupled with a COAG electrode, such that the COAG mode, or both the CUT and COAG modes, may be rendered inoperable upon actuation/opening of thermal fuse **742.** In the embodiment depicted in FIG. 7b, one or more of the electrodes may be positioned in series with thermal fuse **742.** Thermal fuse **742** may then open when a particular threshold temperature is exceeded, thereby blocking the RF energy from reaching the CUT blade or other electrode coupled in series with thermal fuse **742.** In some embodiments, the threshold temperature may comprise a temperature at or near which one or more components of the device are likely to fail and/or at which operation of the device otherwise may become dangerous. FIG. 7C depicts another embodiment of an electrosurgical device **760** comprising a thermal fuse **762** placed in series with a signal line (either a CUT or COAG signal, for example), rather than the actual electrode/RF line. Thus, in the depicted embodiment, thermal fuse **762** is positioned in series
with signal line **770,** which may also be positioned so as to make contact with a multi-stage, sequential switch, as described above, upon actuation. Thermal fuses **742** and **762** are both examples of means for temperature measurement and deactivation. This embodiment may be preferred for certain configurations because it may less susceptible to arcing upon actuation of fuse **762** due to its placement in series with a signal line to the ESU rather than an RF line. Thermal fuse **762** may be positioned in or near tip **764** of TDM **760** or, alternatively, may be positioned within shaft **766** of TDM **760.**

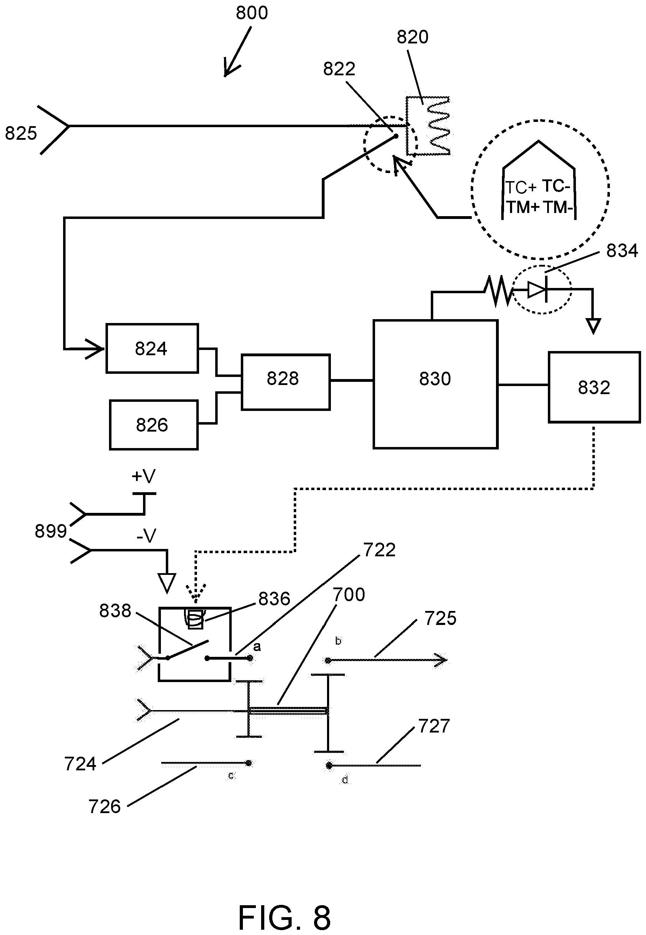

FIG. 8 depicts a schematic diagram of an alternative means for temperature measurement and deactivation. In some embodiments, a temperature sensor **822** may be positioned in or near a tip **820** of a TDM or other electrosurgical device **800** comprising a signal or RF line **825.** Temperature sensor **822** may comprise, for example, a thermistor or thermocouple. As shown in the exploded portion of FIG. 8, the thermistor or thermocouple may comprise a pair of wires (TC+/⁻ or TM+/⁻) which may be coupled to temperature measurement circuitry **824** to convert a microvolt or millivolt figure to, for example, a scaled output represented as millivolts per degrees Celsius. In some embodiments, a limit signal **826** may be used as a reference signal, which may be scaled in the same units as the temperature measurement signal so that, for example, at 200 degrees C the limit signal may be 200mV=0.2V and may trigger a threshold action when the temp measurement output exceeds 0.2V. In other words, when the temperature measurement exceeds the limit signal **826,** a comparator **828** may output a logic level signal to a microcontroller **830,** which may receive an input signal from the comparator **828** and look for the presence of a signal indicative of a temperature having exceeded a threshold temperature. If the comparator **828** receives such a signal indicating that a temperature has exceeded a limit/set point, then microcontroller 830 may activate a relay **832,** which may open a circuit, such as the same circuit that may be opened when a fuse is used in the CUT or COAG control signal path and/or an RF/electrode circuit.

In some embodiments, a visual indicator, such as an LED (Light Emitting Diode) **834** may be coupled with the microcontroller **830,** which may be used to indicate that the device, or at least a portion of the device, has been rendered non-operational to the user due to excessive temperature. The LED **834** may be positioned, for example, on the handle of the device and may indicate to the surgeon that he or she should not keep pressing buttons and/or continue with the procedure.

The relay **832** is normally closed (meaning operational). However, when the microcontroller **830** receives a signal indicating that the threshold temperature has been reached or exceeded, the microcontroller **830** may actuate relay **832** to thereby open one or more circuits to cease operation of the device, or at least one or more elements of the device.

Temperature measurement circuitry **824** may be used, in some embodiments, to compare the actual tip temperature to the temperature limit. If the threshold temperature is exceeded, the relay may be configured to open the "CUT signal" (and/or COAG signal") to the ESU, thereby disabling RF energy. In some embodiments, the energy may be disabled permanently. In other embodiments, the energy may be disabled temporarily while the temperature drops and, optionally, while a fault-condition indicator **834** may alert the user. This circuitry may be powered **899** from a battery source or may be powered by scavenging electrical energy from the ESU, as described in greater detail below. This embodiment may be compatible with the TDM switches described in connection with previous embodiments.

The bottom portion of FIG. 8 illustrates how a signal, such as a relay signal from relay **832,** may be used to open a circuit path to a signal circuit associated with a switch. As illustrated in this portion of the figure, in some embodiments, the circuit path may be opened using, for example, a solenoid **836,** which may be configured to open a circuit upon receiving a signal from, for example, relay **832** by actuating a switch **838.**

In some embodiments, the assembly used to break the circuit upon detection of an excessive temperature may be positioned in series with a signal line, such as line **722** that may be coupled with a "CUT Signal" or "COAG Signal" port of an ESU. A multi-stage, sequential switch, such as switch **700,** as previously described.

In some embodiments, temperature sensor **822** may comprise a bimetallic strip that, when heated, bends and/or breaks contact with one or more RF electrodes and/or the CUT and/or COAG signal wire(s).

Still further embodiments may comprise a temperature sensor **822** that may comprise a phase change sensor. For example, some embodiments may be configured so as to sense a phase change associated with an epoxy or other insulation material, another bonding agent, or another material built into a portion of the TDM (preferably the tip). This phase change material may, in some embodiments, comprise a portion of the device that serves to keep the tip/device together, such as a bonding agent. The bonding agent or other such phase change material may be selected such that a melting temperature of the bonding agent/phase change material is close to a desired threshold temperature for operation of the device.

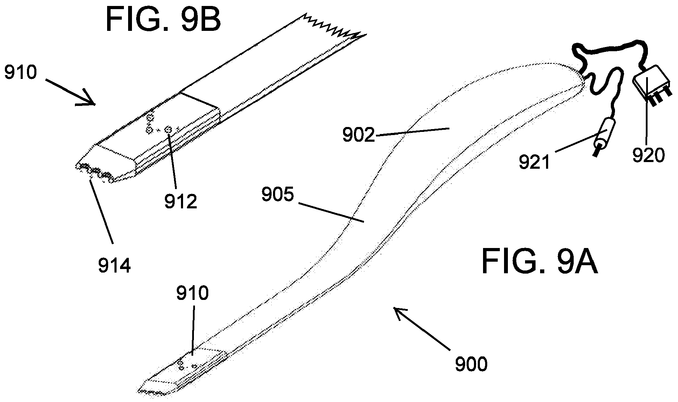

Upon detecting a phase change of the phase change material in the TDM or other electrosurgical device, the TDM may be configured to limit or modulate the energy delivery to one or more of the electrodes. In some embodiments, the TDM may be configured to completely terminate further delivery of RF and/or other energy to some, or all, of the electrodes upon detecting a phase change (likely from solid to liquid). Alternatively, upon detecting a phase change, or detecting that a phase change is likely to happen, a phase change sensor may be configured to reduce, or temporarily suspend, the delivery of RF and/or other energy to some, or all, of the electrodes. In some embodiments, the phase change sensor may be configured to, upon detecting a phase change, or detecting that a phase change is likely to happen, deliver a signal to, for example, the ESG, which may cause the ESG to terminate or modulate, the energy delivery. In some embodiments, the phase change sensor may be configured to, upon detecting a phase change, or detecting that a phase change is likely to happen, sever and/or open one or more of the circuit paths referenced above. In some embodiments, a switch assembly may be configured to provide multiple bipolar electrode sets with separate modes of power delivery. FIG. 9a depicts an example of a TDM 900 configured to allow for switching between distinct bipolar electrode sets. TDM **900** comprises a handle **902,** a tip **910,** a shaft **905** extending between handle **902** and tip **910,** a 3-pinned plug **920,** and a monopolar return connector **921.** The 3-pinned plug **920** may be configured to be positioned into an ESG/ESU receptacle having corresponding pin receptacles. Handle **902** may receive source/signal wires from plug **920** and monopolar return connector **921.** In some embodiments, handle **902** may also house a switching assembly for switching between cutting and coagulation RF waveforms in a plurality of distinct electrode sets, which is another example of a switching means, as described above.

FIG. 9b is a close-up view of tip **910,** which may be made up of a ceramic or other preferably non-conductive material. Tip **910** may comprise a CUT electrode set **914** comprising a plurality of bipolar lysing elements. Such elements may be positioned in between a plurality of protrusions positioned at the distal end of tip **910.** Tip **910** further comprises a COAG electrode set **912,** which may be positioned within an Energy Window on an upper surface of tip **910.** Electrodes **912** terminate at a plurality of bipolar termini in a chevron configuration.

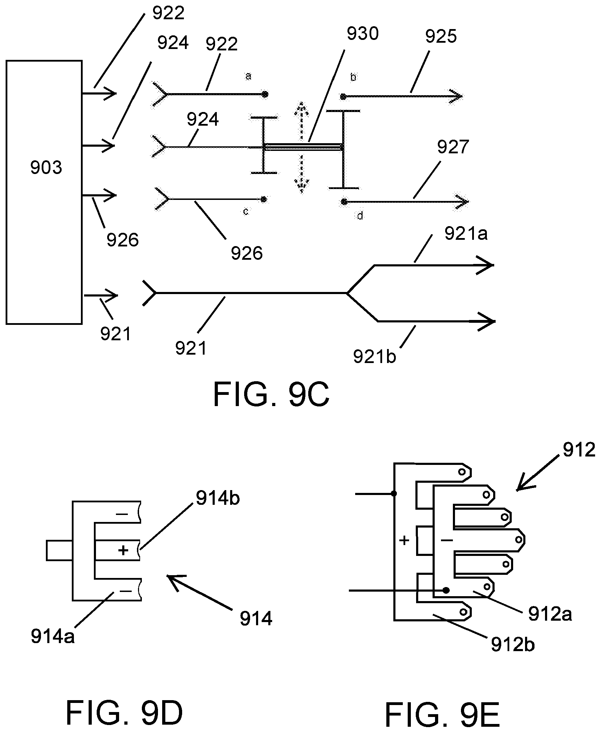

As also shown in FIG. 9b, each electrode in electrode sets **914** and **912** comes in a pair with a corresponding electrode of an opposite polarity, as labeled (+ and -) in the figure. The positive (+) electrodes may receive electrosurgical energy from an ESU via a switch assembly, such as the switch assembly 51 illustrated schematically in Fig. 9c. The negative (-) electrodes may receive electrosurgical energy from an ESU monopolar return via monopolar return connector **921.** The negative conductor of the CUT electrode set **914** and the negative conductor of the COAG electrode set **912** may be connected together and electrically coupled to monopolar return connector **921** at **921a** and **921 b,** respectively. Monopolar return connector may be electrically coupled to a monopolar return of the ESU 903.

The positive conductors of the CUT electrode set **914** may be connected to contact b (FIG. 9c) of switch 930. The positive conductors of the COAG electrode set **912** may be electrically coupled to contact d (FIG. 9c) of switch **930.**

Switch **930** may be operated in a similar manner to the monopolar embodiments described above. Thus, when the switch is enabled for CUT mode, RF will travel to the positive CUT electrodes, pass through tissue and return through the negative electrode and back to the ESU 903 via monopolar return connector **921.** Similarly, when the switch is enabled for COAG mode, RF will travel to the positive COAG electrodes, pass through tissue and return through the negative electrode and back to the ESU via monopolar return connector **921.** More particularly, the top three lines on the left side of the diagram may represent an interface with various typical ports of an ESU **903.** For example, line **922** may be coupled with a "CUT Signal" port 903a of ESU 903, line **924** may be configured to interface with an RF IN port 903b, and line **926** may be configured to interface with a "COAG Signal" port **903c.** Switch **930** may operate, for example, operate by moving switch **930** in one of two directions (up and down from the perspective of the figure). By moving the switch **930** up, the structure of switch **930** may be configured to first electrically couple with contact "b", which may complete a circuit between one or more electrodes (e.g., CUT electrodes) through line **925** and an RF IN port through line **924.** Continued pressing or other movement of switch **930** in the same direction subsequently may result in electrically coupling with contact "a", which may send a CUT signal to the ESU 903 to turn on the CUT mode.

Similarly, by moving the switch **930** in the opposite direction (down from the perspective of the figure), the structure of switch **930** may be configured to first electrically couple with contact "d", which may complete a circuit between one or more electrodes (e.g., COAG electrodes) through line **927** and an RF port through line **924.** Continued pressing or other movement of switch **930** in the same direction may subsequently result in electrically coupling with contact "c", which may send a COAG signal to the ESU to turn on the COAG mode.

Switch **930** may be physically structured to accomplish making these sequential electrical connections automatically upon actuation. For example, in some embodiments, switch 930 may be configured to pivot or flex once contact is made on one side (e.g., with contact b or d) so as to subsequently make contact with a or c, respectively. This may be accomplished by making the contacts on one side of switch **930** longer, as illustrated in FIG. 9c.

FIG. 9d illustrates an example of a possible configuration of CUT electrode set **914,** shown removed from the remainder of TDM 900. The electrodes in set **914** may be arranged such that RF preferentially passes between the activated pair. Thus, positive electrode **914b** may be positioned to extend between opposing ends of negative electrode **914a.** In some embodiments, a dielectric material, such as, for example, a polyimide such as Kaptone, polytetrafluoroethylene, or a suitable ceramic material.

FIG. 9e illustrates an example of a possible configuration of COAG electrode set 912, shown removed from the remainder of TDM 900. The electrodes in set **912** may be arranged such that RF preferentially passes between the activated pair. Thus, positive electrode **912b** may be positioned such that various positive electrode termini extend adjacent to corresponding termini of negative electrode **912a.** As with CUT electrode set **914,** a dielectric material may be used to separate the positive and negative electrodes of COAG electrode set **912.**

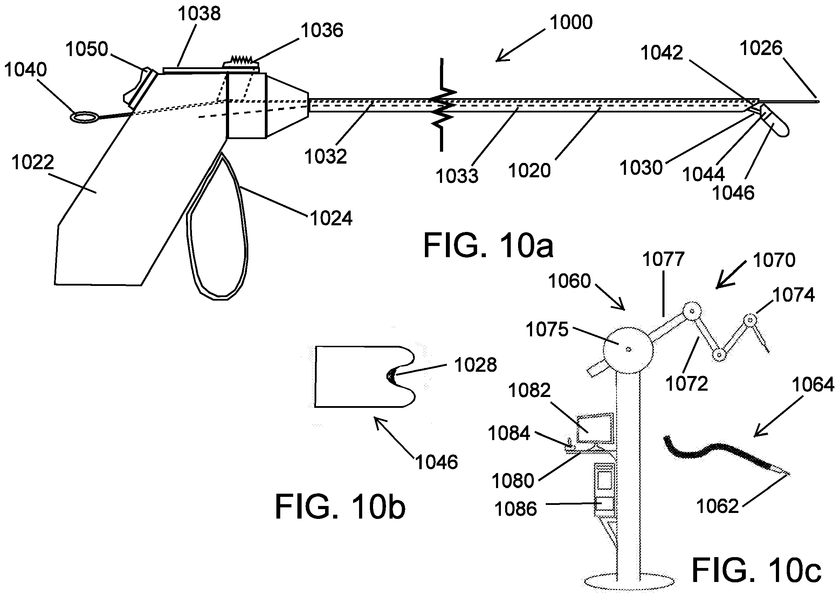

FIG. 10a illustrates another embodiment of a surgical tool **1000** that may be specifically configured for minimally invasive surgery, such as laparoscopic, endoscopic, or keyhole surgery, for example. Tool **1000** comprises a shaft **1020,** a handle **1022,** and a trigger **1024.** A spot coagulator **1032** extends along shaft **1020.** Spot coagulator 1032 may extend adjacent to an exterior surface of shaft **1020.** Alternatively, as depicted in the figure, spot coagulator **1032** may be positioned within a lumen of shaft **1020** and therefore extend within shaft **1020.** Spot coagulator **1032** may comprise a monopolar spot coagulator or, alternatively, may comprise a split tip at spot coagulator tip **1026** and comprise a bipolar spot coagulator. In some embodiments, a water jet **1033** may be positioned to extend adjacent to an exterior surface of shaft **1020** or, alternatively, as depicted in the figure, water jet **1022** may be positioned within a lumen of shaft **1020** and therefore extend within shaft **1020.** Water jet **1022** may comprise a port at a distal end of shaft **1020** to allow for selectively applying a stream of water or another liquid during a surgical procedure.

In some embodiments, shaft **1020** may comprise a Teflon-coated rod made of stainless steel or a similar, preferably biocompatible, material. Shaft **1020** may comprise one or more lumens to allow for various wires, lines, or other items to extend therethrough.

A lysing tip **1046** may be coupled to the distal end of tool **1000.** In some embodiments, lysing tip **1046** may be coupled to the remainder of tool **1000** by using coupling member **1044.** This may allow for use of pre-existing tools, such as laparoscopes, to include various elements for delivery of electrosurgical energy, as described elsewhere herein.

Lysing tip **1046** may comprise one or more lysing elements **1028** configured for delivery of CUT or BLEND electrosurgical energy, as best illustrated in FIG. 10b. In some embodiments, each lysing element **1028** may be positioned between adjacent protrusions formed at the distal end of tip **1046.** Because spot coagulator **1032** may be configured to deliver a different type of electrosurgical energy and/or may be configured to deliver such energy at distinct times, surgical tool **1000** may further comprise a switch assembly **1050** configured to switch between operation of spot coagulator **1032** and lysing element **1028.**

Spot coagulator **1032** may be retractable and/or extendable along lysing tip **1046.** For example, in the depicted embodiment, spot coagulator **1032** may be retracted and/or extended using a spot coagulator moving means, which may comprise toggle **1036.** Toggle 1036 may further comprise a toggle base **1038** to facilitate movement of toggle **1036,** and thereby facilitate movement of spot coagulator **1032,** thereon. In alternative embodiments, various rails, grooves, tracks, ratchets, cables, arms, lines, etc. may be used as spot coagulator moving means. Toggle **1036** may be positioned directly on tool **1000** or, alternatively, may be positioned on a rod, shaft, or other coupling means extending adjacent to tool **1000,** to allow for selective advancement and retraction of the spot coagulator **1032.**

Alternatively, or additionally, a spot coagulator moving means comprising a spot coagulator handle 1040 may be provided. Handle **1040** may comprise a hook, loop, groove, or other feature configured to facilitate use by a finger and/or hand of a surgeon.

A pivoting member **1030** may be coupled with trigger **1024** to allow for pivoting coupling member 1044 and/or lysing tip **1046.** In some embodiments, a shaped laparoscope tip 1042 may be coupled to the distal end of shaft **1020.** Tip **1042** may comprise an angle configured to interface with similar angled proximal edge of coupling member **1044.** As shown in the figure, in some embodiments, the angle at which the proximal edge of coupling member **1044** or, alternatively, a proximal edge of lysing tip **1046,** extends relative to a plane perpendicular to the direction in which shaft **1020** extends may be a mirror image of the distal surface of shaft **1020.** This angle may be selected to facilitate a desired amount of angulation of lysing tip **1046.** In some embodiments, coupling member **1044** may allow for retrofitting a lysing tip **1046** to an existing laparoscope or other surgical instrument.

In some embodiments, one or more of the components discussed above may be coupled with a robotic arm to allow for performing remote surgical procedures. For example, in some embodiments, an assembly comprising a lysing tip, such as lysing tip **1046,** a spot coagulator, such as spot coagulator **1032,** and/or a switch assembly, such as switch assembly 1050, may be coupled with a robotic arm.

Thus, FIG. 10c depicts an embodiment of a system **1060** for performing robotic surgery using an assembly comprising a spot coagulator, switch assembly, and lysing tip. System **1060** may comprise a lysing tip **1062** that may, as described elsewhere herein, comprise a plurality of protrusions with one or more lysing elements positioned therebetween. Lysing tip **1062** may, in some embodiments, be part of an assembly including one or more other components, such as a shaft **1064,** which in some embodiments may comprise a flexible shaft suitable for placement in an endoscope or other similar device. A spot coagulator may be positioned inside of or otherwise adjacent to shaft 1064 and/or lysing tip **1062,** as described above.

In such embodiments, a switch assembly, as described above, may be provided to allow for selective switching between various electrosurgical modes, as described above, for delivery to a lysing element of lysing tip **1062** and/or a spot coagulator. This assembly may be selectively coupled to a robotic arm **1070** such that the alternate means for delivery of electrosurgical energy may be coupled with one or more robotic surgery components to allow a surgeon to perform a surgical procedure with the assembly remotely and/or indirectly. In other embodiments, the assembly may be configured to be integrally coupled with, or otherwise non-selectively coupled with, one or more robotic surgery components. In such embodiments, it may not be necessary to configure the assembly with a handle and/or shaft. In other words, in some embodiments, the assembly may comprise only a tip with a lysing element and spot coagulator. In some embodiments, the robotic surgery system **1060** may comprise one or more motors, such as a screw-drive motor, gear motor, hydraulic motors, etc. In some embodiments, the robotic surgery system **1060** may comprise worm gearheads, video cameras, motor control circuits, monitors, remote control devices, illumination sources, tactile interface, etc. In the embodiment depicted in FIG. 10c, robotic arm **1070** comprises a plurality of arm segments **1072** with corresponding joints **1074** positioned therebetween. A primary joint **1075** may be positioned to support and articulate together each of the arm segments **1072** and smaller joints **1074.** Primary joint **1075** has a primary arm segment **1077** that extends therefrom. Finer movements of the robotic arm may then be accomplished using one or more of the smaller joints **1076.** A stand **1080** may also be provided to support the various robotic arms. In some embodiments, stand **1080** may also be configured to support a monitor **1082** and/or other display, input, or control components, such as a control element **1084.** In some embodiments, control element **1084** may comprise a hand control toggle. In other embodiments, control element **1084** may comprise a keyboard, mouse, touchscreen display, virtual reality system, control pad, or the like. Monitor **1082** and/or control element **1084** may be communicatively coupled with a central processing unit **1086.**

## Claims

1. An electrosurgical device (120), comprising:
a first electrode set (133) configured to deliver CUT radiofrequency energy;
a second electrode set (134) configured to deliver COAG radiofrequency energy; and
a switch assembly (150) configured to allow for selection between at least three modes to facilitate operation of the electrosurgical device (120) in the at least three modes, the at least three modes comprising:
a first, neutral mode, in which the electrosurgical device (120) is configured such that no radiofrequency energy is delivered to either the first electrode set (133) or the second electrode set (134);
a second, CUT mode, in which the electrosurgical device (120) is configured such that CUT radiofrequency energy may be delivered to the first electrode set (133) through the switch assembly (150); and
a third, COAG mode, in which the electrosurgical device (120) is configured such that COAG radiofrequency energy may be delivered to the second electrode set (134) through the switch assembly (150);
**characterized in that** the switch assembly (150) is further configured to allow for selection of a first sub-mode of the CUT mode in which an electrical path between the CUT electrode set (133) and a radiofrequency source is closed but an electrical path associated with a signal circuit (181B, 181C) for activating the radiofrequency source is open.

2. The electrosurgical device (120) of claim 1, wherein the switch assembly (150) comprises a rocker switch (250).

3. The electrosurgical device (120) of claim 1, wherein the switch assembly (150) is configured to automatically transition from the first sub-mode of the CUT mode to a final configuration of the CUT mode in which the electrical path associated with the signal circuit (181B, 181C) for activating the radiofrequency source is closed upon actuation of the switch assembly (150).

4. The electrosurgical device (120) of claim 1, wherein the switch assembly (150) is further configured to allow for selection of a first sub-mode of the COAG mode in which an electrical path between the COAG electrode set (134) and a radiofrequency source is closed but an electrical path associated with a signal circuit (181B, 181C) for activating the radiofrequency source is open.

5. The electrosurgical device (120) of claim 4, wherein the switch assembly (150) is configured to automatically transition from the first sub-mode of the COAG mode to a final configuration of the COAG mode in which the electrical path associated with the signal circuit (181B, 181C) for activating the radiofrequency source is closed upon actuation of the switch assembly (150).

6. The electrosurgical device (120) of claim 5, wherein the switch assembly (150) is further configured to effect at least one of:
open the electrical path between the COAG electrode set (134) and the radiofrequency source; and
increase a clearance distance between adjacent contacts (252) associated with the COAG electrode set (134);
upon transitioning between the neutral mode and the CUT mode.

7. The electrosurgical device (120) of claim 6, wherein the switch assembly (150) is further configured to effect at least one of:
open the electrical path between the CUT electrode set (133) and the radiofrequency source; and
increase a clearance distance between adjacent contacts (252) associated with the CUT electrode set (133);
upon transitioning between the neutral mode and the COAG mode.

8. The electrosurgical device (120) of claim 1, wherein the switch assembly (150) comprises a reed switch (624, 625).

9. The electrosurgical device (120) of claim 8, wherein the switch assembly (150) comprises a first reed switch configured to selectively open and close an electrical path to the first electrode set (133) and a second reed switch configured to selectively open and close an electrical path for activating a signal circuit (181B) for activating the CUT radiofrequency energy.

10. The electrosurgical device (120) of claim 1, wherein the switch assembly (150) is further configured such that, in the first mode, an electrical path between the first electrode set (133) and a radiofrequency source is open and an electrical path between the second electrode set (134) and a radiofrequency source is open.

11. The electrosurgical device (120) of claim 1, wherein the switch assembly (150) is further configured such that, in the first mode, an electrical path between the first electrode set (133) and a radiofrequency source is closed, an electrical path between the second electrode set (134) and a radiofrequency source is closed, an electrical path associated with a signal circuit (181B, 181C) for activating the CUT radiofrequency energy is open, and an electrical path associated with a signal circuit (181C) for activating the COAG radiofrequency energy is open.

12. The electrosurgical device (120) of claim 11, wherein the switch assembly (150) is configured to open the electrical path between the first electrode set (133) and the radiofrequency source upon transitioning from the first mode to the third mode, and wherein the switch assembly (150) is further configured to open the electrical path between the second electrode set (134) and the radiofrequency source upon transitioning from the first mode to the second mode.

## Patentansprüche

1. Elektrochirurgische Vorrichtung (120), umfassend:
einen ersten Elektrodensatz (133), der konfiguriert ist, um SCHNEID-Hochfrequenzenergie zu liefern;
einen zweiten Elektrodensatz (134), der konfiguriert ist, um KOAG-Hochfrequenzenergie zu liefern; und
eine Schalteranordnung (150), die konfiguriert ist, um die Auswahl zwischen wenigstens drei Modi zu ermöglichen, um den Betrieb der elektrochirurgischen Vorrichtung (120) in den wenigstens drei Modi zu erleichtern, wobei die wenigstens drei Modi umfassen:
einen ersten neutralen Modus, in dem die elektrochirurgische Vorrichtung (120) derart konfiguriert ist, dass weder dem ersten Elektrodensatz (133) noch dem zweiten Elektrodensatz (134) Hochfrequenzenergie zugeführt wird;
einen zweiten SCHNEID-Modus, in dem die elektrochirurgische Vorrichtung (120) derart konfiguriert ist, dass die SCHNEID-Hochfrequenzenergie durch die Schalteranordnung (150) an den ersten Elektrodensatz (133) geliefert werden kann; und
einen dritten KOAG-Modus, in dem die elektrochirurgische Vorrichtung (120) derart konfiguriert ist, dass KOAG-Hochfrequenzenergie durch die Schalteranordnung (150) an den zweiten Elektrodensatz (134) geliefert werden kann;
**dadurch gekennzeichnet, dass** die Schalteranordnung (150) ferner konfiguriert ist, um die Auswahl eines ersten Untermodus des SCHNEID-Modus zu ermöglichen, in dem ein elektrischer Pfad zwischen dem SCHNEID-Elektrodensatz (133) und einer Hochfrequenzquelle geschlossen ist, jedoch ein elektrischer Pfad, der einem Signalstromkreis (181B, 181C) zugeordnet ist, für die Aktivierung der Hochfrequenzquelle offen ist.

2. Elektrochirurgische Vorrichtung (120) nach Anspruch 1, wobei die Schalteranordnung (150) einen Wippschalter (250) umfasst.

3. Elektrochirurgische Vorrichtung (120) nach Anspruch 1, wobei die Schalteranordnung (150) konfiguriert ist, um automatisch von dem ersten Untermodus des SCHNEID-Modus zu einer endgültigen Konfiguration des SCHNEID-Modus überzugehen, in dem der elektrische Pfad, der der Signalschaltung (181B, 181C) zugeordnet ist, für die Aktivierung der Hochfrequenzquelle bei der Betätigung der Schalteranordnung (150) geschlossen ist.

4. Elektrochirurgische Vorrichtung (120) nach Anspruch 1, wobei die Schalteranordnung (150) ferner konfiguriert ist, um die Auswahl eines ersten Untermodus des KOAG-Modus zu ermöglichen, in dem ein elektrischer Pfad zwischen dem KOAG-Elektrodensatz (134) und einer Hochfrequenzquelle geschlossen ist, jedoch ein elektrischer Pfad, der einem Signalstromkreis (181B, 181C) zugeordnet ist, zum Aktivieren der Hochfrequenzquelle offen ist.

5. Elektrochirurgische Vorrichtung (120) nach Anspruch 4, wobei die Schalteranordnung (150) konfiguriert ist, um automatisch von dem ersten Untermodus des KOAG-Modus zu einer endgültigen Konfiguration des KOAG-Modus überzugehen, in dem der elektrische Pfad, der der Signalschaltung (181B, 181C) zugeordnet ist, für die Aktivierung der Hochfrequenzquelle bei der Betätigung der Schalteranordnung (150) geschlossen ist.

6. Elektrochirurgische Vorrichtung (120) nach Anspruch 5, wobei die Schalteranordnung (150) ferner konfiguriert ist, um zu bewirken:
Öffnen des elektrischen Pfads zwischen dem KOAG-Elektrodensatz (134) und der Hochfrequenzquelle; und/oder
Erhöhen eines Sicherheitsabstands zwischen benachbarten Kontakten (252), die dem KOAG-Elektrodensatz (134) zugeordnet sind;
beim Übergehen zwischen dem neutralen Modus und dem SCHNEID-Modus.

7. Elektrochirurgische Vorrichtung (120) nach Anspruch 6, wobei die Schalteranordnung (150) ferner konfiguriert ist, um zu bewirken:
Öffnen des elektrischen Pfads zwischen dem SCHNEID-Elektrodensatz (133) und der Hochfrequenzquelle; und/oder
Erhöhen eines Sicherheitsabstands zwischen benachbarten Kontakten (252), die dem SCHNEID-Elektrodensatz (133) zugeordnet sind;
beim Übergehen zwischen dem neutralen Modus und dem KOAG-Modus.

8. Elektrochirurgische Vorrichtung (120) nach Anspruch 1, wobei die Schalteranordnung (150) einen Reedschalter (624, 625) umfasst.

9. Elektrochirurgische Vorrichtung (120) nach Anspruch 8, wobei die Schalteranordnung (150) einen ersten Reedschalter, der zum selektiven Öffnen und Schließen eines elektrischen Pfades zu dem ersten Elektrodensatz (133) konfiguriert ist, und einen zweiten Reedschalter umfasst, der zum selektiven Öffnen und Schließen eines elektrischen Pfads zum Aktivieren einer Signalschaltung (181B) für das Aktivieren der SCHNEID-Hochfrequenzenergie konfiguriert ist.

10. Elektrochirurgische Vorrichtung (120) nach Anspruch 1, wobei die Schalteranordnung (150) ferner derart konfiguriert ist, dass in dem ersten Modus ein elektrischer Pfad zwischen dem ersten Elektrodensatz (133) und einer Hochfrequenzquelle offen ist und ein elektrischer Pfad zwischen dem zweiten Elektrodensatz (134) und einer Hochfrequenzquelle offen ist.

11. Elektrochirurgische Vorrichtung (120) nach Anspruch 1, wobei die Schalteranordnung (150) ferner derart konfiguriert ist, dass in dem ersten Modus ein elektrischer Pfad zwischen dem ersten Elektrodensatz (133) und einer Hochfrequenzquelle geschlossen ist, ein elektrischer Pfad zwischen dem zweiten Elektrodensatz (134) und einer Hochfrequenzquelle geschlossen ist, ein elektrischer Pfad, der einem Signalstromkreis (181B, 181C) zugeordnet ist, für die Aktivierung der SCHNEID-Hochfrequenzenergie offen ist und ein elektrischer Pfad, der einem Signalstromkreis (181C) zugeordnet ist, für die Aktivierung der KOAG-Hochfrequenzenergie offen ist.

12. Elektrochirurgische Vorrichtung (120) nach Anspruch 11, wobei die Schalteranordnung (150) konfiguriert ist, um den elektrischen Pfad zwischen dem ersten Elektrodensatz (133) und der Hochfrequenzquelle beim Übergehen von dem ersten Modus in den dritten Modus zu öffnen, und wobei die Schalteranordnung (150) ferner konfiguriert ist, um den elektrischen Pfad zwischen dem zweiten Elektrodensatz (134) und der Hochfrequenzquelle beim Übergehen von dem ersten Modus in den zweiten Modus zu öffnen.

## Revendications

1. Dispositif électrochirurgical (120), comprenant :
un premier ensemble d'électrodes (133) conçu pour délivrer une énergie radiofréquence CUT ;
un second ensemble d'électrodes (134) conçu pour délivrer une énergie radiofréquence COAG ; et
un montage de commutateur (150) conçu pour permettre une sélection entre au moins trois modes pour faciliter le fonctionnement du dispositif électrochirurgical (120) dans les au moins trois modes, les au moins trois modes comprenant :
un premier mode neutre, dans lequel le dispositif électrochirurgical (120) est conçu de sorte qu'aucune énergie radiofréquence ne soit délivrée au premier ensemble d'électrodes (133) ou au second ensemble d'électrodes (134) ;
un deuxième mode CUT, dans lequel le dispositif électrochirurgical (120) est conçu de sorte que l'énergie radiofréquence CUT puisse être délivrée au premier ensemble d'électrodes (133) par l'intermédiaire du montage de commutateur (150) ; et
un troisième mode COAG, dans lequel le dispositif électrochirurgical (120) est conçu de sorte qu'une énergie radiofréquence COAG puisse être délivrée au second ensemble d'électrodes (134) par l'intermédiaire du montage de commutateur (150) ;
**caractérisé en ce que** le montage de commutateur (150) est en outre conçu pour permettre la sélection d'un premier sous-mode du mode CUT dans lequel un chemin électrique entre l'ensemble d'électrodes CUT (133) et une source de radiofréquences est fermé mais un chemin électrique associé à un circuit de signal (181B, 181C) pour activer la source de radiofréquence est ouvert.

2. Dispositif électrochirurgical (120) selon la revendication 1, ledit montage de commutateur (150) comprenant un commutateur à bascule (250).

3. Dispositif électrochirurgical (120) selon la revendication 1, ledit montage de commutateur (150) étant conçu pour effectuer automatiquement une transition du premier sous-mode du mode CUT à une configuration finale du mode CUT dans laquelle le chemin électrique associé au circuit de signal (181B, 181C) pour activer la source radiofréquence est fermé lors de l'actionnement du montage de commutateur (150).

4. Dispositif électrochirurgical (120) selon la revendication 1, ledit montage de commutateur (150) étant en outre conçu pour permettre la sélection d'un premier sous-mode du mode COAG dans lequel un chemin électrique entre l'ensemble d'électrodes COAG (134) et une source radiofréquence est fermé mais un chemin électrique associé à un circuit de signal (181B, 181C) pour activer la source radiofréquence est ouvert.

5. Dispositif électrochirurgical (120) selon la revendication 4, ledit montage de commutateur (150) étant conçu pour effectuer automatiquement une transition du premier sous-mode du mode COAG à une configuration finale du mode COAG dans lequel le chemin électrique associé au circuit de signal (181B, 181C) pour activer la source radiofréquence est fermé lors de l'actionnement du montage de commutateur (150).

6. Dispositif électrochirurgical (120) selon la revendication 5, ledit montage de commutateur (150) étant en outre conçu pour effectuer au moins l'un de :
l'ouverture du chemin électrique entre l'ensemble d'électrodes COAG (134) et la source radiofréquence ; et
l'augmentation d'une distance de jeu entre les contacts adjacents (252) associés à l'ensemble d'électrodes COAG (134) ;
lors de la transition entre le mode neutre et le mode CUT.

7. Dispositif électrochirurgical (120) selon la revendication 6, ledit montage de commutateur (150) étant en outre conçu pour effectuer au moins l'un de :
l'ouverture du chemin électrique entre l'ensemble d'électrodes CUT (133) et la source radiofréquence ; et
l'augmentation d'une distance de jeu entre des contacts adjacents (252) associés à l'ensemble d'électrodes CUT (133) ;
lors de la transition entre le mode neutre et le mode COAG.

8. Dispositif électrochirurgical (120) selon la revendication 1, ledit montage de commutateur (150) comprenant un commutateur à lames (624, 625).

9. Dispositif électrochirurgical (120) selon la revendication 8, ledit montage de commutateur (150) comprenant un premier commutateur à lames conçu pour ouvrir et fermer sélectivement un chemin électrique vers le premier ensemble d'électrodes (133) et un second commutateur à lames conçu pour ouvrir et fermer sélectivement un chemin électrique pour activer un circuit de signal (181B) pour activer l'énergie radiofréquence CUT.

10. Dispositif électrochirurgical (120) selon la revendication 1, ledit montage de commutateur (150) étant en outre conçu de sorte que, dans le premier mode, un chemin électrique entre le premier ensemble d'électrodes (133) et une source radiofréquence soit ouvert et un chemin électrique entre le second ensemble d'électrodes (134) et une source radiofréquences soit ouvert.

11. Dispositif électrochirurgical (120) selon la revendication 1, ledit montage de commutateur (150) étant en outre conçu de sorte que, dans le premier mode, un chemin électrique entre le premier ensemble d'électrodes (133) et une source radiofréquence soit fermé, un chemin électrique entre le second ensemble d'électrodes (134) et une source radiofréquences soit fermé, un chemin électrique associé à un circuit de signal (181B, 181C) pour activer l'énergie de radiofréquence CUT soit ouvert, et un chemin électrique associé à un circuit de signal (181C) pour activer l'énergie radiofréquence COAG soit ouvert.

12. Dispositif électrochirurgical (120) selon la revendication 11, ledit montage de commutateur (150) étant conçu pour ouvrir le chemin électrique entre le premier ensemble d'électrodes (133) et la source radiofréquences lors de la transition du premier mode au troisième mode, et ledit montage de commutateur (150) étant en outre conçu pour ouvrir le chemin électrique entre le second ensemble d'électrodes (134) et la source radiofréquences lors de la transition du premier mode au deuxième mode.
